Europäisches Patentamt

(19) European Patent Office

Office européen des brevets

(11) **EP 0 420 085 B1**

(12) **EUROPÄISCHE PATENTSCHRIFT**

(45) Veröffentlichungstag und Bekanntmachung des Hinweises auf die Patenterteilung:
**01.07.1998 Patentblatt 1998/27**

(51) Int. Cl.$^6$: **A61B 5/021**, A61B 5/022, A61B 5/0456

(21) Anmeldenummer: **90118241.0**

(22) Anmeldetag: **22.09.1990**

(54) **Messung der Herzfunktion**

Measuring heart function

Mesure de la fonction cardiaque

(84) Benannte Vertragsstaaten:
**AT BE CH DE DK ES FR GB GR IT LI LU NL SE**

(30) Priorität: **27.09.1989 IL 91803**
**10.08.1990 US 565642**

(43) Veröffentlichungstag der Anmeldung:
**03.04.1991 Patentblatt 1991/14**

(73) Patentinhaber: **Pyrotec Limited**
**Tortola (BVI) (VG)**

(72) Erfinder: **Pearlman, Andrew L.**
**D.N. Misgav 20164 (IL)**

(74) Vertreter:
**Döring, Wolfgang, Dr.-Ing.**
**Patentanwälte**
**Hauck, Graalfs, Wehnert, Döring, Siemons et al**
**Mörickestrasse 18**
**40474 Düsseldorf (DE)**

(56) Entgegenhaltungen:
WO-A-82/01122          WO-A-87/00414

- CIRCULATION. Bd. 67, Nr. 4, April 1983, Seiten 844 - 853; MAGORIEN ET AL.: 'Assessment of left ventricular pressure-volume relations using gated radionuclide angiography, echocardiography, and micromanometer pressure recordings.'
- INTERNATIONAL CONFERENCE ON BIOMEDICAL TRANSDUCERS. Bd. 2, November 1975, PARIS, FR Seiten 377 - 382; HAGER ET AL.: 'Système automatisé non-envahissant mesurant la pente de la pression systolique pour la détection des maladies vasculaires périphériques.'
- Clinical Cardiology,no. 10, Marmor et al.: "Method for Noninvasive Measurement of Central Aortic Systolic Pressure", pages 215-221 (1987).

EP 0 420 085 B1

## Beschreibung

Die vorliegende Erfindung betrifft ein Verfahren zum nichtinvasiven Messen des Herzleistungsindex als Maß für die Herzfunktion unter Ruhe- und Bewegungsbedingungen und eine Vorrichtung zur Durchführung eines derartigen Verfahrens.

Gemäß dem Stand der Technik sind verschiedenartige Herzmonitore bekannt. Die bekannten Monitore machen üblicherweise von Messungen Gebrauch, die mit Hilfe eines Eingriffes unter Herzkatheterisierung oder ohne Eingriff durchgeführt werden. Ein Verfahren zum nichtinvasiven Messen des systolischen Drucks der zentralen Aorta ist in einem Artikel "Method for Noninvasive Measurement of Central Aortic Systolic Pressure" von A. Marmor et al., Clinical Cardiology, 1987 beschrieben. Mit diesem Verfahren werden Kurven des systolischen Drucks der zentralen Aorta erzeugt. Das Verfahren basiert auf der Darstellung der Okklusivdruckwerte, mit denen die Armarterie beaufschlagt wird, in Abhängigkeit von den Zeitintervallen, die für den Ausgleich und Durchbruch der Druckwelle der Aorta durch den Okklusivdruck der Armarterie benötigt werden. Die entsprechenden Zeitintervalle werden erhalten, indem man die Zeit vom Beginn der Depolarisation (QRS-Komplex) bis zur Detektion der Druckwelle an der zeitweise verschlossenen Armarterie mißt.

In dem Artikel wird ferner beschrieben, wie durch Vergleiche festgestellt wurde, daß mit diesem Verfahren tatsächlich der Druck der zentralen Aorta oder eine enge Annäherung an diesen Druck gemessen wird. Es wird ferner erläutert, daß durch Kombination von derartigen nichtinvasiven Druckmessungen mit Volumenmessungen, die durch Radionuklid-Angiographie erhalten werden, Druck-Volumen-Kurven erstellt werden. Es wird eine mögliche klinische Anwendung dieser Druck-Volumen Kurven vorgeschlagen.

Der Erfindung liegt die Aufgabe zugrunde, ein Verfahren und eine Vorrichtung zum nichtinvasiven Messen eines Parameters für die Herzfunktion unter Ruhe- und Bewegungsbedingungen zur Verfügung zu stellen.

Diese Aufgabe wird erfindungsgemäß durch ein Verfahren zum nichtinvasiven Messen des Herzleistungsindex als Maß für die Herzfunktion unter Ruhe- und Bewegungsbedingungen gelöst, das die folgenden Schritte umfaßt:

nichtinvasives Messen des Drucks der linken Herzkammer in Abhängigkeit von der Zeit durch Messen der Ankunftszeiten von Herzdruckimpulsen an einer vorgegebenen arteriellen Stelle im Abstand vom Herz bei einer Vielzahl von Manschettendruckwerten;

nichtinvasives Messen des Volumens der linken Herzkammer in Abhängigkeit von der Zeit;

Bestimmen der Arbeit der linken Herzkammer als Produkt aus dem Druck der linken Herzkammer und dem Volumen der linken Herzkammer in Abhängigkeit von der Zeit;

Bestimmen der Leistung der linken Herzkammer als Ableitung dieses Produktes nach der Zeit; und

Bestimmen der Steigung dieser zeitlichen Ableitung in der Anstiegsphase während des Intervalls vom Beginn der Systole bis zum Augenblick maximaler Leistung als Wert für den Herzleistungsindex.

In Weiterbildung der Erfindung umfaßt der Schritt des Messens des Drucks der linken Herzkammer das Konzentrieren der größten Zahl der Druckmessungen in dem Intervall während der frühen Ausstoßphase der linken Herzkammer.

Gemäß einer bevorzugten Ausführungsform der Erfindung ist das Verfahren weiter dadurch gekennzeichnet, daß der Schritt des Messens des Drucks der linken Herzkammer ferner den Schritt des Messens der Ankunftszeiten von Herzdruckimpulsen an der vorgegebenen Stelle während der Zeitdauer umfaßt, während der der Druck der linken Herzkammer von 100% auf 125% des enddiastolischen Wertes ansteigt.

Das Verfahren umfaßt auch den Schritt der Anzeige eines Realzeit-Elektrokardiogrammes und von Blutdruckwellen auf einer kontinuierlich aktualisierten Basis.

Gemäß einer bevorzugten Ausführungsform der Erfindung ist das Verfahren ferner dadurch gekennzeichnet, daß es die Anzeige der Druckwerte der linken Herzkammer und der entsprechenden volumetrischen Werte der linken Herzkammer gleichzeitig und zusammen mit dem Elektrokardiogramm und den Blutdruckwellen umfaßt.

Gemäß einer weiteren bevorzugten Ausführungsform der Erfindung ist das Verfahren dadurch gekennzeichnet, daß es den Schritt der Messung der Ankunftszeit für einen vorgegebenen Sperrdruck während eines oder mehreren Herzzyklen und der Speicherung der gemessenen Zeiten für jeden Druck umfaßt.

Gemäß einer anderen Ausführungsform der vorliegenden Erfindung umfaßt der Schritt der Messung der Ankunftszeit den Schritt der Zurückweisung von Zeitwerten, die eine nicht akzeptable Varianz besitzen.

Ferner umfaßt bei einer bevorzugten Ausführungsform der Erfindung der Schritt des Messens der Ankunftszeit ebenfalls den Schritt der statistischen Durchschnittsbildung von diversen annehmbaren Stichprobenpunkten, um die Effekte der Varianz von Herzschlag zu Herzschlag, von Störsignalen und des Verrauschens herabzusetzen.

Gemäß einer weiteren Ausführungsform der Erfindung enthält der Schritt des Messens des Volumens der linken Herzkammer die Durchführung von mindestens einer Messung innerhalb von 15 msec des QRS-Komplexes.

Ferner enthält gemäß einer Ansführungsform der Erfindung der Schritt des Messens des Volumens der linken Herzkammer die Durchführung einer Vielzahl von Volumenmessungen innerhalb von 40 msec.

Darüber hinaus ist bei einer Ansführungsform der Erfindung das Verfahren weiter durch die Messung des systolischen und diastolischen Blutdrucks gekennzeichnet.

Gemäß einer bevorzugten Ansführungsform der Erfindung wird ferner der Herzleistungsindex als Steigung der mit der Methode der kleinsten Quadrate ermittelten besten Regressionsanpassungskurve an eine Reihe von momentanen Leistungswerten bis zu einem maximalen Leistungspunkt, ausgenommen die Punkte, deren Werte außerhalb des Varianzbereiches liegen, der den anderen Punkten entspricht, errechnet.

In Weiterbildung der Erfindung wird in Anpassung an die Ankunftszeiten bei der Vielzahl der Druckwerte eine Kurve gezogen, die sich der Zeitabhängigen Welle des Drucks der linken Herzkammer annähert.

Eine besonders bevorzugte Ausführungsform des erfindungsgemäßen Verfahrens besteht in der Messung der Ankunftszeiten der Herzimpulse durch Messung der Doppler des Blutstromes an der vorgegebenen Stelle. Hierzu finden ein spezieller Doppler-Ultraschallsensor und Prozessor Verwendung, die im nachfolgenden Text beschrieben werden.

Das erfindungsgemäße Verfahren besitzt den Vorteil, daß die Herzfunktion in zuverlässiger Weise im Bewegungs- bzw. Stresszustand des Patienten gemessen werden kann. Dies wird insbesondere durch das Doppler-Blutstrommeßverfahren zusammen mit einer sehr speziellen Verarbeitung der empfangenen Doppler-Signale erreicht, was zu einer klaren und rauschfreien Charakterisierung der Herzleistung bzw. Herzfunktion, d.h. der Druck- und Volumen-Zeit-Kurve oder Druck-Zeit-Kurve, fuhrt.

Die vorstehend angegebene Aufgabe wird ferner durch eine Vorrichtung zum Durchführen des vorstehend beschriebenen Verfahrens gelöst mit

Einrichtungen zum nichtinvasiven Messen des Drucks der linken Herzkammer in Abhängigkeit von der Zeit durch Messen der Ankunftszeiten von Herzdruckimpulsen an einer vorgegebenen arteriellen Stelle im Abstand vom Herz bei einer Vielzahl von Manschettendruckwerten;

Einrichtungen zum nichtinvasiven Messen des Volumens der linken Herzkammer in Abhängigkeit von der Zeit;

Einrichtungen zum Bestimmen der Arbeit der linken Herzkammer als Produkt aus dem Druck der linken Herzkammer und dem Volumen der linken Herzkammer in Abhängigkeit von der Zeit;

Einrichtungen zum Bestimmen der Leistung der linken Herzkammer als Ableitung dieses Produktes nach der Zeit; und

Einrichtungen zum Bestimmen der Steigung dieser zeitlichen Ableitung in der Anstiegsphase während des Intervalls vom Beginn der Systole bis zum Augenblick maximaler Leistung als Wert für den Herzleistungsindex.

Gemäß einer Ansführungsform der Erfindung umfassen die Einrichtungen zum Messen des Drucks der linken Herzkammer des weiteren Einrichtungen zum Konzentrieren der größten Zahl der Druckmessungen im Intervall während der frühen Ausstoßphase.

Gemäß einer Ausführungsform der Erfindung ist die Vorrichtung ferner dadurch gekennzeichnet, daß die Einrichtungen zum Messen des Drucks der linken Herzkammer auch Einrichtungen zum Messen der Ankunftszeiten der Herzdruckimpulse an der vorgegebenen Stelle während einer Zeitdauer umfaßt, während der der Druck der linken Herzkammer von 100% auf 125% des enddiastolischen Wertes ansteigt.

Ferner umfaßt bei einer Ausführungsform der Erfindung die Vorrichtung Einrichtungen zum Anzeigen eines Realzeit-Elektrokardiogrammes und von Blutdruck-Wellen auf einer kontinuierlich aktualisierten Basis.

Gemäß einer bevorzugten Ausführungsform der vorliegenden Erfindung ist die Vorrichtung des weiteren dadurch gekennzeichnet, daß sie Einrichtungen zum Anzeigen der Druckwerte der linken Herzkammer und der errechneten entsprechenden volumetrischen Werte der linken Herzkammer gleichzeitig und zusammen mit dem Elektrokardiogramm und den Blutdruckwellen aufweist.

Gemäß einer bevorzugten Ausführungsform der vorliegenden Erfindung ist die Vorrichtung ferner dadurch gekennzeichnet, daß sie Einrichtungen zum Messen der Ankunftszeit für einen vorgegebenen Absperrdruck während eines oder mehreren Herzzyklen und zum Speichern der gemessenen Zeiten für jeden Druck enthält.

Bei einer bevorzugten Ausführungsform der vorliegenden Erfindung umfassen die Einrichtungen zum Messen der Ankunftszeit Einrichtungen zum Zurückweisen von Zeitwerten, die eine nicht akzeptierbare Varianz besitzen.

Auch umfassen gemäß einer Ausführungsform der vorliegenden Erfindung die Einrichtungen zum Messen der Ankunftszeit Einrichtungen zur statistischen Durchschnittsbildung von diversen annehmbaren Stichprobenpunkten, um die Auswirkungen der Varianz von Schlag auf Schlag, von Störsignalen und des Verrauschens herabzusetzen.

Ferner umfassen gemäß einer bevorzugten Ausführungsform der vorliegenden Erfindung die Einrichtungen zum Messen des Volumens der linken Herzkammer Einrichtungen zum Durchführen von mindestens einer Messung innerhalb 15 msec des QRS.

Gemäß einer bevorzugten Ausführungsform der vorliegenden Erfindung besitzen die Einrichtungen zum Messen des Volumens der linken Herzkammer Einrichtungen zum Ausführen einer Vielzahl von Volumenmessungen innerhalb von 40 msec.

Ferner besitzt gemäß einer bevorzugten Ausführungsform der vorliegenden Erfindung die Vorrichtung Einrichtungen zum Messen des systolischen und diastolischen Blutdrucks.

Ferner umfaßt gemäß einer bevorzugten Ausführungsform der vorliegenden Erfindung die Vorrichtung auch einen Impulswellensensor und/oder Impulswellenprozessor mit reduzierter Bewegungsbeeinflussung zur Verfügung gestellt. Gemäß einer bevorzugten Ausführungsform der Erfindung handelt es sich bei den Einrichtungen zum Erfassen der Ankunft der Herzdruckwellen an einer vorgegebenen arteriellen Stelle, vorzugsweise einer Stelle der Arterie des Armes, um einen Doppler-Ultraschall-Arterienwandbewegungssensor.

Gemäß einer anderen besonders bevorzugten Ausführungsform der erfindungsgemäßen Vorrichtung handelt es sich bei den Einrichtungen zum Erfassen der Ankunft der Herzdruckwelle an einer vorgegebenen arteriellen Stelle, vorzugsweise einer Stelle der Arterie des Armes, um einen Doppler-Ultraschall-Blutstromsensor. Der Sensor selbst und eine entsprechende, damit kombinierte Verarbeitungseinheit ermöglichen die Zurückweisung von Bewegungseinflüssen.

Der Doppler-Ultraschallsensor (Wandler) wird vorteilhafterweise von einem Armband gehalten, das einen verstellbaren Träger für den Wandler umfaßt, der an einem verstellbaren Befestigungsstreifen fixiert ist. Der Doppler-Ultraschallsensor (Wandler) ist vorzugsweise als flache Packung ausgebildet, wobei die Doppler-Kristalle so montiert sind, daß sie einen festen Neigungswinkel zur Horizontalen besitzen, der typischerweise 30° beträgt.

Der Impulswellenprozessor enthält vorzugsweise ein Hochpassfilter, das die Hochfrequenzen vom Audiosignal trennt, und einen RMS-Amplituden-Gleichstromumformer, der die Energie des Hochfrequenzspektrums mißt und den gesamten RMS (quadratischen Mittelwert) in eine proportionale Gleichspannung umwandelt.

Die Erfindung wird nachfolgend anhand von Ausführungsbeispielen in Verbindung mit der Zeichnung im einzelnen erläutert. Es zeigen:

Figur 1 ein Funktionsblockdiagramm eines Herleistungsmonitors (CPIM), der gemäß einer bevorzugten Ausführungsform der vorliegenden Erfindung ausgebildet ist;

Figur 2 eine Ausführung des Systems auf der Basis der Ausführungsform der Figur 1;

die Figuren 3A, 3B und 3C die Ableitung von Punkten auf einer Druck-Zeit-Kurve unter Verwendung einer Manschette, eines Elektrokardiographen und eines distalen Impulswellenformsensors;

die Figuren 4A, 4B und 4C idealisierte Diagramme eines Elektrokardiogrammes, des Drucks der Armarterie und der Wandbewegung der Armarterie als Funktion der Zeit, die für das Verständnis der Funktionsweise der Vorrichtung der Figur 1 von Bedeutung sind;

Figur 5 eine mögliche Version eines Manschettendrucksteueralgorithmus zur optimalen Erniedrigung des Manschettendrucks;

die Figuren 6A, 6B und 6C die Aquisition und Synchronisation von Volumen-Druck-Kurven und die Berechnung der sich ergebenden Herzfunktionskurve, aus der der Herzleistungsindex (CPI) abgeleitet wird;

Figur 7 eine spezielle Ausführungsform eines Impulswellenformsensors zusammen mit einer Halteeinrichtung;

Figur 8 eine weitere Ausführungsform der Halteeinrichtung für den Impulswellenformsensor;

Figur 9 ein Blockdiagramm eines Prozessors für den Impulswellenformsensor;

Figur 10 ein exaktes Schaltbild des Prozessors gemäß Figur 9;

| Figur 11 | ein Blockdiagramm einer Manschettendrucksteuereinheit; und |
|---|---|
| Figur 12 | ein exaktes Schaltbild der Manschettendrucksteuereinheit gemäß Figur 11. |

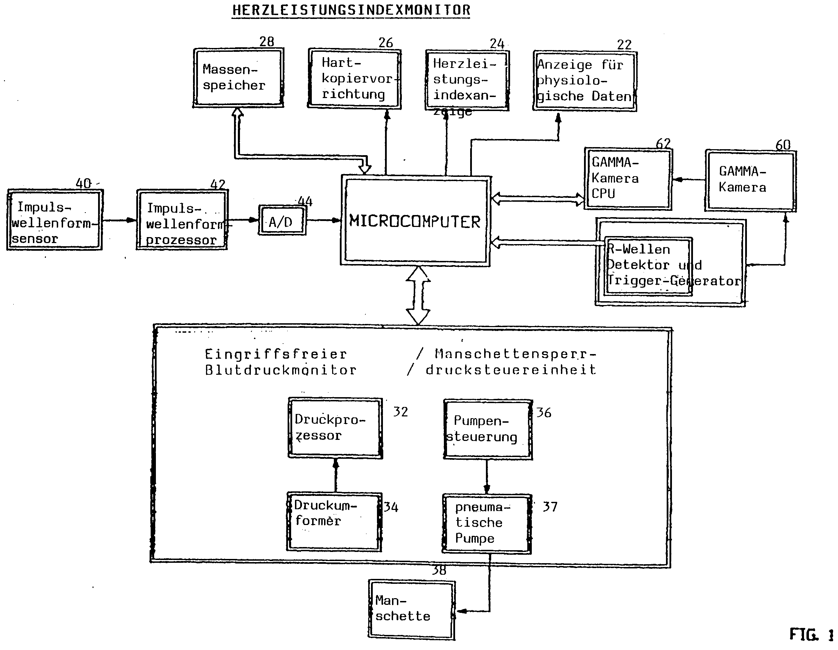

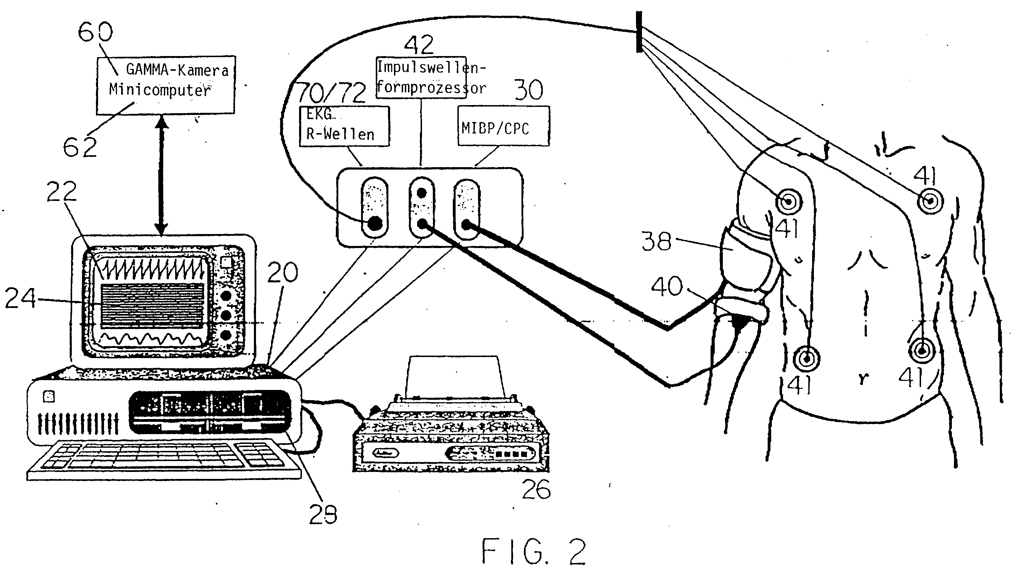

Es wird nunmehr auf Figur 1 Bezug genommen, die in Blockdarstellung einen Herzleistungsindexmonitor zeigt, der gemäß der vorliegenden Erfindung ausgebildet ist. Es wird ferner auf Figur 2 Bezug genommen, die eine Ausführung eines Systems auf der Basis der Ausführungsform der Figur 1 zeigt. Der mit 10 bezeichnete Herzmonitor umfaßt einen Mikrocomputer 20, der vorzugsweise IBM-PC kompatibel ist. Der Mikrocomputer 20 steuert vorzugsweise sämtliche Monitorfunktionen und betreibt eine Anzeige 22 für physiologische Daten, beispielsweise einen EGA-Grafikvideomonitor, und eine Anzeige 24 für den Herzleistungsindex (CPI), der von der gleichen Vorrichtung, die für die Anzeige 22 verwendet wird, vorgesehen werden kann. Der Mikrocomputer 20 speichert ferner Daten in einer Massenspeichervorrichtung 28 und gewinnt Daten aus dieser. Hierbei handelt es sich vorzugsweise um einen Hartplattenantrieb mit mindestens 10 mBytes. Der Mikrocomputer betreibt ferner eine Hartkopiervorrichtung 26, vorzugsweise einen Epson kompatiblen Punktmatrixdrucker.

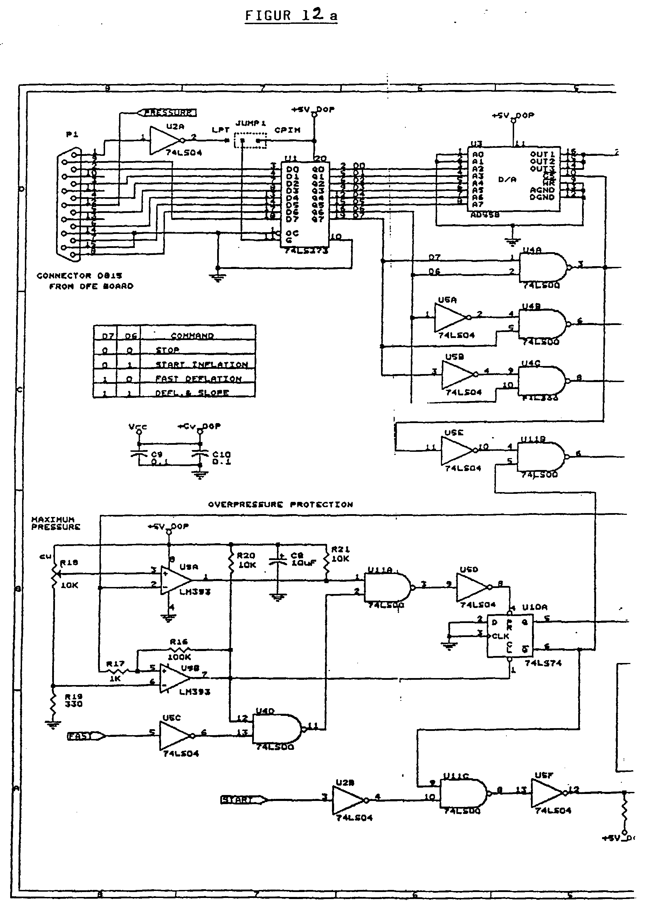

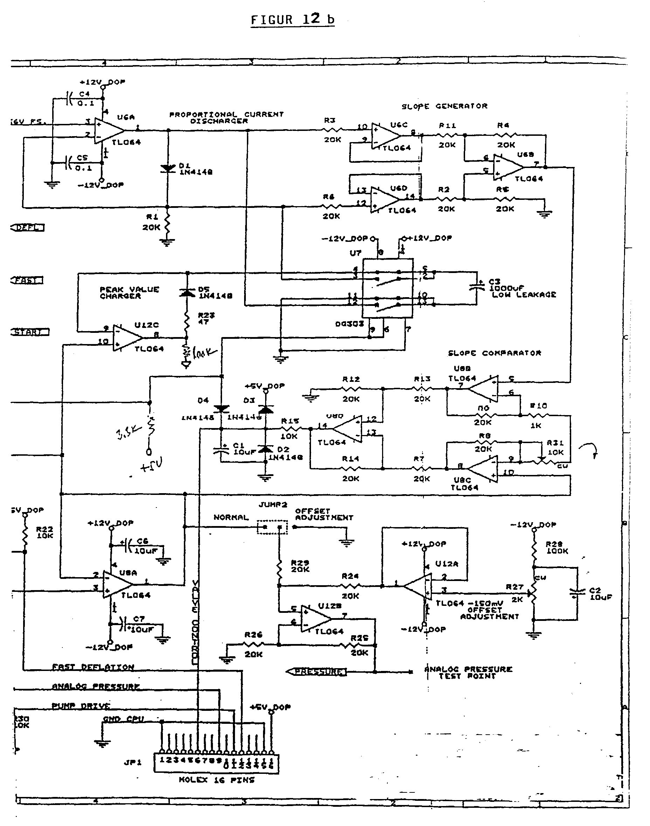

Der Monitor der Figur 1 umfaßt ferner eine Vorrichtung 30 zur eingriffsfreien Blutdruckmessung (NIBP) und zur Manschettendrucksteuerung (CPC), wie beispielsweise eine Bosch EBM 502D, zum Messen des Druckes der Armarterie und der Herzfrequenz, die eine sphygmomanometrische Manschette 38 betätigt. Bei der Manschette 38 handelt es sich vorzugsweise um eine Wickelmanschette, wie sie im Pedisphyg-System von Cas Medical, Inc., Branford, Connecticut, USA, verwendet wird, oder um eine Bosch-Manschette. Die Manschettendrucksteuervorrichtung umfaßt geeignete Schnittstellen und Steuerschaltungen sowie Software, um eine Funktionsweise der Vorrichtung 30 zur Drucksteuerung der Manschette 38 anstelle ihrer herkömmlichen Betriebsweise zur Blutdruckmessung zu ermöglichen. Ein Blockdiagramm der Steuervorrichtung ist in Figur 12 gezeigt.

Der Monitor 10 umfaßt ferner einen EKG-Monitor 70 und einen R-Wellen-Detektor sowie Triggergenerator 72, die beide üblicherweise bei standardmäßigen EKG-Monitorsystemen vorhanden sind, wie beispielsweise einem Mennen Horizon 2000 Patientenmonitor.

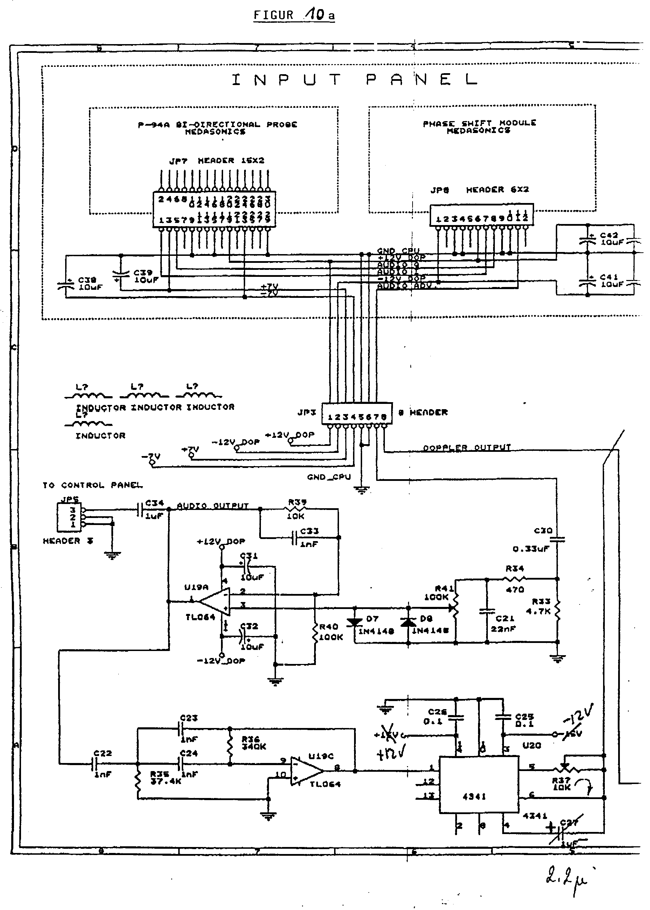

Ferner ist im Monitor 10 ein Impulswellenformsensor 40, nämlich ein Doppler-Ultraschallwandbewegungs- und -blutstromerfassungssensor, wie beispielsweise ein Meda Sonics Modell 94G, enthalten, der am gleichen Arm befestigt ist wie die Manschette 38, und zwar in einer Entfernung von etwa 1-3 cm zur Manschette. Ein Impulswellenformprozessor 42 (gezeigt in Figur 10), vorzugsweise eine Analog und/oder Digital-Schaltung, deren Eingangssignal die Wellenform vom Sensor 40 darstellt, stellt ein analoges Ausgangssignal zur Verfügung, das vorzugsweise zum Blutstrom proportional ist.

Alternativ dazu kann das Ausgangssignal zur Wandbewegung oder zur Geschwindigkeit der Wandbewegung proportional sein. In jeder Ausführungsform eliminieren Hochpassfilter den größten Teil von Bewegungseinflüssen vom Ausgangssignal zum A/D-Wandler 44, dessen digitales Ausgangssignal vom Mikrocomputer 20 gelesen wird.

Eine Gamma-Kamera 60, bei der es sich um eine im Handel befindliche Gesichtsfeld-Gamma-Kamera handeln kann, wie beispielsweise ein Elscint Model APEX, mit zugehöriger CPU 62 empfängt ein R-Wellenauslösesignal entweder von einem EKG-Monitor 70 oder von ihrem eigenen internen EKG-Monitor. In Abhängigkeit davon zeichnet die Kamera 60 eine Vielzahl von Rahmen von einigen msec Dauer in Intervallen von üblicherweise 25-40 msec über jeden Herzzyklus auf und bildet einen Durchschnittswert aus den Rahmen von vielen (üblicherweise 300) Zyklen, um die durchschnittlichen volumetrischen Rahmenwerte entlang der Zeitkurve über den Herzzyklus zu erhalten.

Eine CPU 62 der Gamma-Kamera überträgt die resultierenden Datenwerte über eine digitale Verbindung, vorzugsweise RS232 oder Centronics, parallel oder alternativ über Platten auf den Mikrocomputer 20.

Wie in Figur 2 gezeigt, ist die Manschette 30 vorzugsweise über einem Ellbogen befestigt und wird vom Mikrocomputer 20 über die Manschettendrucksteuervorrichtung 30 gesteuert. Ein R-Wellen-Detektor und Triggergenerator 72 erfaßt die scharfe Spitze der Welle des EKG, bekannt als QRS-Komplex, und stellt einen digitalen Auslöseimpuls zur Verfügung, der dem Auftreten der R-Welle (dem Mittelpunkt der QRS-Spitze) entspricht.

Die Herzleistung wird als die zeitliche Ableitung des Produktes aus dem Herzvolumen und dem Herz-(oder Aorta-) Druck mit der Zeit definiert. Der Herzleistungsindex wird als die Steigung des Abschnittes der Leistung-Zeit-Kurve vom Beginn der Systole bis zum Moment maximaler Leistung definiert.

Hiernach wird die Ermittlung der Herzleistungskurve und des Herzleistungsindex (CPI) unter Verwendung des Herzmonitors 10 beschrieben.

Schätzung des Drucks der linken Herzkammer

Durch Absperren des Blutstromes durch den Arm während des größten Teiles des Herzzyklus wird eine stehende Flüssigkeitssäule zwischen der Aorta und der Armarterie erzeugt, so daß die Wellenform des ansteigenden Drucks innerhalb der Aorta mit minimaler Verzerrung auf die Armarterie übertragen wird. Die an der Armarterie erhaltenen Druckwerte repräsentieren daher stark angenähert die in der linken Herzkammer.

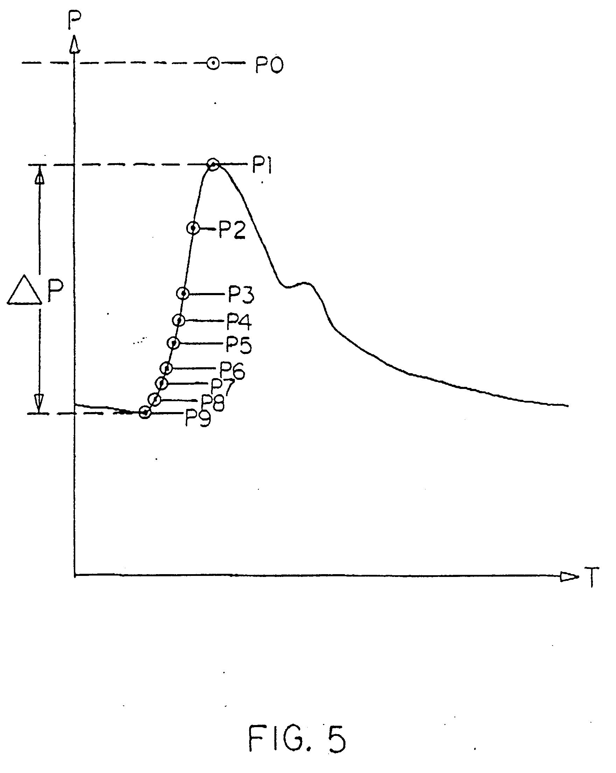

Um eine spätere Kombination mit am Herz durchgeführten Volumenmessungen der linken Herzkammer zu ermöglichen, müssen die Armdruckwerte zeitlich verschoben werden, um der Fortpflanzung der Herzdruckwelle vom Herz zur Armarterie Rechnung zu tragen. Die Zeit nach dem QRS-Komplex, die für eine Herzdruckwelle zur Bewegung vom Herz zur Meßstelle an der Armarterie erforderlich ist, wird hier als Fortpflanzungszeit bezeichnet, wie nachfolgend in Verbindung mit Figur 5 beschrieben wird. Die Fortpflanzungszeit für einen bestimmten Patienten während der Prüfungsdauer wird unter sämtlichen Bedingungen der Herztätigkeit als konstant angesehen.

Die Funktionsweise des Herzmonitors 10 einschließlich der Berechnung des CPI ist im Ablaufdiagramm am Schluß der Beschreibung erläutert. Die Vorbereitungen des Patienten zur Ventrikulographie mit der Gamma-Kamera werden beendet, und 3-4 EKG-Elektroden 41 werden in üblicher Thorax-Anbringung befestigt, so daß sie der EKG-Vorrichtung 70 Eingangssignale liefern. Während sich der Patient im Ruhezustand befindet, wird die Manschette 39 unmittelbar über einem Ellbogen angelegt, und der Impulswellenformsensor 40 wird in einer Entfernung von 1-3 cm von der Manschette am gleichen Arm angebracht. Das Impulswellenformsignal wird vom Mikrocomputer 20 von der Vorrichtung 42 angefordert und zusammen mit dem EKG auf der Anzeige 22 für die physiologischen Daten angezeigt. Die Qualität der EKG- und Imnpulswellenform-Signale wird als visuelle Rückkopplung benutzt, um eine richtige Signalerzeugung zu verifizieren oder um irgendwelche erforderlichen Einstellungen zu führen.

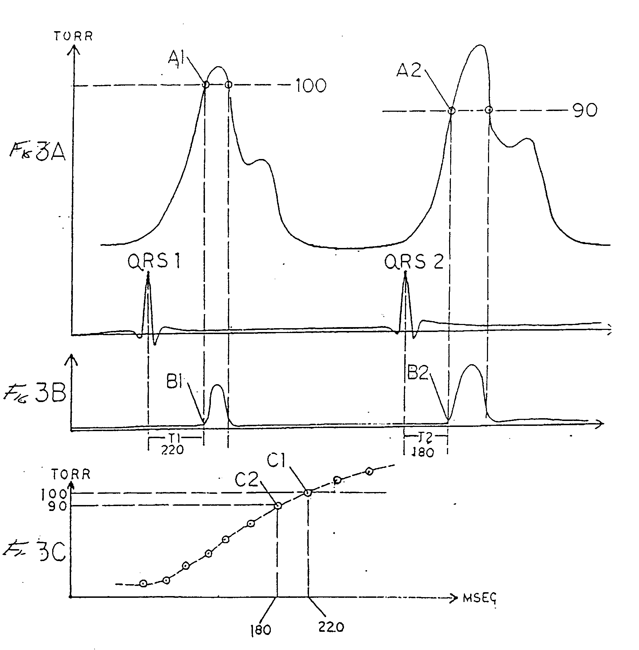

Die Figuren 3A, 3B und 3C stellen die Art und Weise dar, in der die Stichprobenpunkte auf der Druck-Zeit-Kurve bestimmt werden, und zwar über die Beziehung zwischen dem Armarteriendruck, dem Manschettendruck, dem EKG QRS-Komplex und der Erfassung einer Impulswellenform im Abstand von der Manschette.

Es sind zwei vereinfachte Herzzyklen mit repräsentativen Parametern in den Figuren 3A-3C dargestellt. Im ersten Herzzyklus beträgt der systolische Druck 110 Torr, und der Manschettendruck wurde auf 100 Torr eingestellt, während im zweiten Zyklus der systolische Druck 115 betrug und der Manschettendruck auf 90 eingestellt wurde. In Figur 3A sind die Wellenform des Armdrucks, der Manschettendruck und die EKG-Wellenform dargestellt, wobei die entsprechende Abstimmung des QRS-Komplexes eines jeden Herzzyklus und die resultierende Armdruckwellenform gezeigt sind.

Der Punkt A1 des Herzzyklus 1 tritt zum ersten Mal während des Zyklus auf, wenn der Armdruck den Manschettendruck übersteigt. Aus Figur 3B, die die Impulswellenform zeigt, die vom Impulswellenformprozessor 42 erzeugt wird, geht hervor, daß die Impulswellenform am Punkt B1 abrupt ansteigt. Punkt B1 fällt zeitlich mit Punkt A1 in Figur 3Aa zusammen, wenn die Blutdruckwelle die Manschette passiert, d.h. durchbricht, und erzeugt eine Arterienwandbewegung, die von der Vorrichtung 42 erfaßt wird.

Die zeitliche Verzögerung vom QRS-Komplex bis zum Beginn des abrupten Anstiegs der Impulswellenform ist mit T1 bezeichnet und besitzt eine Größe von 220 msec. Sie stellt in Figur 3B die Zeit nach dem QRS-Komplex dar, in der der Druck der Armarterie 100 Torr erreicht hat. In Figur 3C, in der die Druck-Zeit-Kurve dargestellt ist, besitzt der Punkt C1 einen Druckwert von 100 Torr und eine Zeit von 220 msec gemäß den Druck- und Zeitwerten der Punkte A1 und B1. Der Zeitmaßstab der Figur 2C ist in msec, während der der Figuren 3A und 3B in sec ist.

In analoger Weise entsprechen dem Herzzyklus 2, bei dem der systolische Druck 115 Torr und der Manschettendruck 90 Torr betragen, die Pukte A2 und B2 der Zeit, wenn die Blutdruckwelle die Manschette durchbricht, was 180 msec nach dem QRS-Komplex des Herzzyklus 2 auftritt. In Figur 3C besitzt der Punkt C2 einen Druck von 90 Torr und eine Zeit von 180 msec gemäß den Druck- und Zeitwerten der Punkte A2 und B2. Bei der tatsächlichen Ausführungsform wird jeder Punkt der Druck-Zeit-Kurve bestimmt, indem man den Durchschnittswert aus den für einen bestimmten Manschettendruck, der über eine Vielzahl von Herzzyklen aufrechterhalten wird, gemessenen Verzögerungszeiten bildet.

Während sich der Patient noch in der Ruhelage befindet, bewirkt die Bedienungsperson die Initialisierung des Herzmonitors 10 für den Meßbeginn. Während der Initialisierung und vor dem Anlegen eines Druckwertes an der Manschette 38 wird die Fortpflanzungszeit des Arteriendrucks vom Herz zur Armarterie geschätzt und die Impulswellenform charakterisiert.

Der Herzmonitor 20 wird betätigt, um die maximalen und minimalen Impulswellenformwerte zu messen. Die Impulswellenformwerte MAXAMP und MINAMP stellen die entsprechenden durchschnittlichen Maximal- und Minimalwerte der Impulswellenformausgangssignale des Detektors 42 während einer Vielzahl von Herzzyklen, vorzugsweise 10, dar. MAXAMP wird vorzugsweise erhalten, indem man vom maximalen Amplitudenwert des Aussgangssignales des Detektors 42 aus der vorstehend erwähnten Vielzahl von Herzzyklen den Durchschnittswert bildet, während MINAMP vorzugsweise erhalten wird, indem man von dem minimalen Amplitudenwert des Ausgangssignales des Detektors 42 aus jedem der vorstehend erwähnten Vielzahl von Herzzyklen den Durchschnittswert bildet.

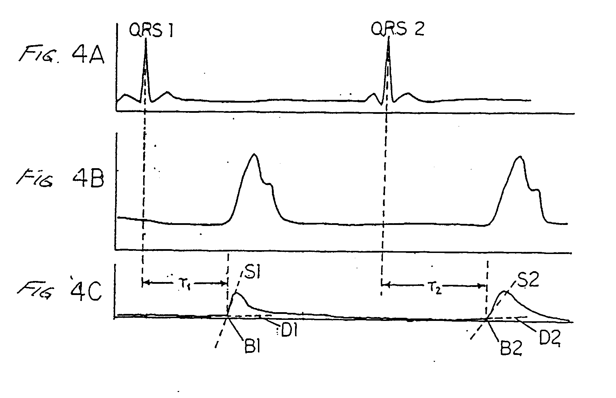

Die Figuren 4A, 4B und 4C zeigen ein Verfahren zum Berechnen der Fortpflanzungszeit, das ebenfalls zum Berechnen der Durchbruchszeit, auf die nachfolgend Bezug genommen wird, verwendet wird. Die Figuren 4A, 4B und 4C zeigen die EKG-Wellenform, die Armarteriendruckwellenform und die Impulswellenform für zwei idealisierte Herzzyklen. Die Fortpflanzungszeit wird errechnet, indem man zuerst den steigenden Aufschwung der in Figur 4C gezeigten Impulswellenform erfaßt.

Eine Regressionslinie, die im ersten Zyklus mit S1 und im zweiten Zyklus mit S2 bezeichnet ist, wird an den ersten Abschnitt der Aufschwungphase angepaßt, vorzugsweise an die Punkte von den ersten 30 msec der Aufschwungphase an. Eine zweite Regressionslinie, die mit D1 im ersten Zyklus und mit D2 im zweiten Zyklus bezeichnet ist, wird an den letzten Abschnitt der Wellenform vor dem Aufschwung angepaßt, und zwar vorzugsweise an die Punkte während der letzten 30 msec vor dem Aufschwung. Das Zeitintervall T1 von der R-Welle des QRS-Komplexes 1 bis zum Schnittpunkt B1 zwischen den Linien S1 und Da gibt die Ankunftszeit der Impulswelle des Herzzyklus 1 am Impulswellenformsensor 40 wieder. In entsprechender Weise kennzeichnet das Zeitintervalal T2 von der R-Welle des QRS-Komplexes 2 bis zum Punkt B2 die Ankunftszeit der Impulswelle des Herzzyklus 2 am Sensor 40. Wenn man die Fortpflanzungszeit ermittelt, wird vorzugsweise aus den vorstehend genannten Ankunftszeiten aus einer Vielzahl von Herzzyklen, vorzugsweise 10 Zyklen, der Durchschnittswert gebildet.

Die Bedienungsperson bewirkt dann, daß die Vorrichtung 30 den diastolischen und systolischen Druckwert und die Herzfrequenz über den Mikrocomputer 20 erhält. Ein Manschettendrucksteueralgorithmus, von dem eine Ausführungsform in Figur 5 dargestellt ist, verwendet die gemessenen diastolischen und systolischen Druckwerte und wählt die Drücke aus, auf die die Manschette aufgeblasen werden soll.

Bevorzugt wird die Reihe der Druckwerte, die von der Manschette 31 realisiert werden soll, so definiert, daß die größte Zahl der Druckmessungen während der frühen Ausstoßphase, die üblicherweise als die Phase zwischen 100-125% des enddiastolischen Druckes definiert wird, konzentriert wird. Ein Beispiel für einen Optimierungsalgorithmus zur Definition der Druckwerte ist in Figur 5 dargestellt, wobei die Drücke PO bis P9 wie folgt eingestellt werden:

Für DP - systolischer Druck - diastolischer Druck
PO - 1,25 • systolischer Druck
P1 - systolischer Druck
P2 - systolischer Druck - 0,25 • DP
P3 - systolischer Druck - 0,50 • DP
P4 - systolischer Druck - 0,65 • DP
P5 - systolischer Druck - 0,75 • DP
P6 - systolischer Druck - 0,85 • DP
P7 - systolischer Druck - 0,90 • DP
P8 - systolischer Druck - 0,95 • DP
P9 - diastolischer Druck

Die Zahl der Punkte und ihre genaue Abhängigkeit vom systolischen und diastolischen Druck kann vom vorstehenden abweichen, solange wie eine Vielzahl von Punkten im Druckbereich vom enddiastolischen Punkt bis zur Mitte des systolischen Anstiegs, d.h. vom diastolischen Druck bis zu (systolischer Druck - 0,5 • DP) vorhanden ist. In Abhängigkeit von einem Befehl der Bedienungsperson an den Monitor 10 wird die Manschette 38 auf den Druck PO aufgeblasen, und das Impulsdetektorausgangssignal dazu verwendet, um die Absperrung des Blutstromes durch die Manschette zu verifizieren.

Der Schwellenwert für die Bestätigung der Absperrung ist dann erreicht, wenn die Ausgangsamplitude des Impulswellenformdetektors 42 geringer ist als ein Bruchteil der Differenz zwischen den vorstehend erwähnten Werten MAXAMP und MINAMP, vorzugsweise 0,05 • (MAXAMP - MINAMP). Wenn der ursprüngliche Manschettendruck PO den Ausgang des Detektors 42 in obenstehender Weise nicht reduziert, wird der Wert PO angehoben, vorzugsweise um 10% seines vorherigen Wertes, und der Bestätigungsvorgang wird wiederholt. Das wird solange wiederholt, bis die Absperrung bestätigt ist oder bis der Wert PO ein Maximum von 150% des systolischen Druckes erreicht. Wenn die Absperrung einmal bestätigt ist, wird aus den erfaßten Impulswellenformwerten über eine Vielzahl von Herzzyklen, typischerweise 10, ein Durchschnittswert gebildet, um einen durchschnittlichen Basislinienwert AMP zu erhalten.

Die Bedienungsperson betätigt dann den Monitor 10, um mit der Messung der Druck-Zeit-Kurve zu beginnen. Der Manschettendruck wird auf den Wert P1 reduziert, damit ein Durchbruch nur in der Nähe der systolischen Spitze auftritt. Der Mikrocomputer 20 analysiert das Druckwellenformsignal in Realzeit während des laufenden Herzzyklus, um festzustellen, wenn und wann der Durchbruch auftritt. Als Durchbruch wird üblicherweise der Punkt definiert, an dem der Wellenformwert zuerst beträchtlich über die Basislinie ansteigt, was bei der bevorzugten Ausführungsform einem Anstieg von mehr als drei Standardabweichungen über den vorstehend erwähnten durchschnittlichen Basislinienwert AMP entspricht.

Wenn ein Durchbruch erfaßt wird, findet das vorstehend beschriebene Verfahren zur Bestimmung der Fortpflanzungszeit zur Schätzung der Durchbruchszeit Verwendung. Dieses Verfahren wird während mindestens 2, typischerweise 5-10, Herzzyklen für die gleiche Manschettendruckeinstellung wiederholt, woraus mindestens 2, typischerweise 5-10, Schätzungen der Durchbruchszeit für den Druck resultieren, aus denen der Mittelwert und die Varianz für die Durchbruchszeit errechnet werden. Bevor zu einem neuen Manschettendruckwert übergegangen wird, wird die Reihe der Durchbruchszeitschätzungen durchgesehen und, außerhalb liegende Werte (typischerweise diejenigen, die mehr als drei Standardabweichungen vom Mittelwert entfernt liegen) werden aus der Reihe ausgeschlossen, und es wird ein neuer endgültiger Mittelwert errechnet. Dieser endgültige Mittelwert wird in der Druck-Volumen-Kurve für den eingesetzten Manschettendruckwert gespeichert.

Wenn der endgültige Druck-Zeit-Punkt für einen vorgegebenen Manschettenwert einmal ermittelt worden ist, wird der Manschettendruck dann auf den nächsten im Manschettendrucksteueralgorithmus bestimmten Wert reduziert, bis der letzte Wert erreicht worden ist.

Aus der Betrachtung der Figur 3 geht hervor, daß bei niedrigen Drücken, beispielsweise solchen, die nahe am diastolischen Druck liegen, das vorstehend erwähnte Verfahren unzuverlässig sein kann, da die erforderliche stehende Blutsäule vor dem Beginn der Systole nicht gut aufgebaut ist. Daher wird der Druck-Zeit-Wert für den Beginn der Systole als jüngster gemessener diastolischer Druckwert genommen, und dessen Zeit wird als die vorstehend erwähnte Fortpflanzungszeit gewählt, die im Ruhezustand des Patienten ermittelt wurde.

Die auf diese Weise erhaltene Reihe von Druckwerten wird üblicherweise durch stückweise polynomische Kurvenanpassung mit Hilfe einer Minimierung nach der Methode der kleinsten Quadrate interpoliert, um geschätzte Druckwerte bei jedem beliebigen gewünschten Zeitpunkt während des systolischen Abschnittes des Herzzyklus vorzusehen. Die in Figur 6B gezeigte Druckkurve, die üblicherweise dem Durchschnitt von Druckwerten entspricht, die über eine Vielzahl von Herzzyklen aufgezeichnet worden sind, wie vorstehend erwähnt, wird dann um die Größe der Fortplanzungsverzögerung verschoben, so daß auf diese Weise eine geschätzte Druckkurve für die linke Herzkammer ermittelt wird.

Bestimmung des Volumens der linken Herzkammer Es wird nunmehr wiederum auf Figur 1 Bezug genommen. Wie vorstehend erwähnt, umfaßt bei der bevorzugten Ausführungsform die Erfindung zusätzlich eine Gesichtsfeld-Gamma-Kamera 60, wie sie beispielsweise von der Firma Elscint aus Haifa, Israel erhältlich ist, mit zugehöriger CPU 62. Die Gamma-Kamera 60 und die CPU 62 messen mit Hilfe der Radionuklid-Ventrikulographie nach dem Zählverfahren das Volumen der linken Herzkammer. Dieses Verfahren ist in der Veröffentlichung "Left Ventricular Pressure-Volume Diagrams and End-systolic Pressure-Volume Relations in Human Beings", von McKay, R.G. et al., die im Journal of the American College of Cardiology, Vol. 3, 1984 publiziert wurde, beschrieben.

Gemäß einer bevorzugten Ausführungsform der Erfindung erfaßt der R-Wellendetektor 72 die R-Welle des EKG-Signales. Wenn alternativ dazu die Gamma-Kamera 72 eine EKG-Vorrichtung und einen zugehörigen QRS oder R-Wellendetektor umfaßt, wird das QRS-Signal oder die R-Welle vom Detektor der Gamma-Kamera erfaßt.

Eine vorgegebene Zeitspanne später, typischerweise 10-20 msec, zählt die Gamma-Kamera 60 die Zahl der Gammastrahlen, die von der linken Herzkammer kommen, während eines vorgegebenen Zeitrahmens, typischerweise 5-10 msec. Die Gamma-Kamera 60 wiederholt die Messung alle 20-50 ms und erzeugt Punkte auf einer Kurve des Volumens der linken Herzkammer über die Zeit. Die auf diese Weise erzeugte Volumenkurve wird über den R-Wellendetektor auf den QRS-Komplex synchronisiert und ist in Figur 6A dargestellt.

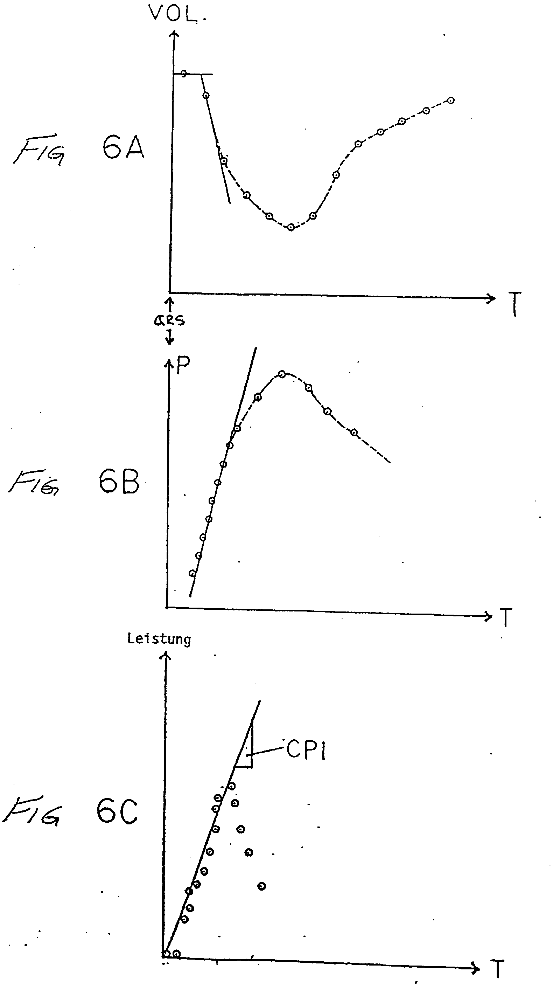

Typischerweise besitzt diese Volumenkurve nur einige Punkte und wird somit durch stückweise polynomische Kurvenerstellungsverfahren mit Hilfe der Methode der kleinsten Quadrate interpoliert. Es wird auf diese Weise eine interpolierte Volumenkurve, wie in Figur 6A gezeigt, errechnet, die zu den gleichen Zeitpunkten wie die mit der vorstehend beschriebenen Methode errechnete Druckkurve Daten besitzt. Die Herzleistungskurve kann somit aus der Volumenkurve und der Druckkurve errechnet werden, wie in den Figuren 6A, 6B und 6C gezeigt.

Berechnung der Herzleistungskurve und des CPI-Faktors

In bezug auf eine Vielzahl von Punkten über die Systole, normalerweise 32 Punkte, wird das Produkt aus den entsprechenden Druck- und Volumenwerten berechnet. Die Ableitung des Produktes nach der Zeit wird unter Einsatz eines Differenzierverfahrens zweiter Ordnung näherungsweise bestimmt, um entsprechende Punkte auf einer Herzleistungskurve zu ermitteln, die in Figur 6C gezeigt ist. Bei der bevorzugten Ausführungsform wird der CPI-Faktor aus den Herzleistungskurvenwerten in der folgenden Weise errechnet:

Eine lineare Regressionslinie wird an die Punkte der Leistungskurve zwischen dem Beginn der Systole und dem Maximalwert einschließlich desselben angepaßt. Jedwede Datenpunkte, deren Werte um mehr als zwei Standardabweichungen von der linearen Regressionslinie entfernt liegen, werden ausgeschlossen. Nach dem Ausschließen der außerhalb liegenden Punkte wird eine neue Regressionslinie errechnet, und deren Steigung wird als endgültiger CPI-Wert benutzt.

Die gesamte Sequenz der Funktionsweise des Monitors 10, wie sie vorstehend beschrieben wurde, ist im Ablaufdiagramm am Ende der Beschreibung zusammengefaßt.

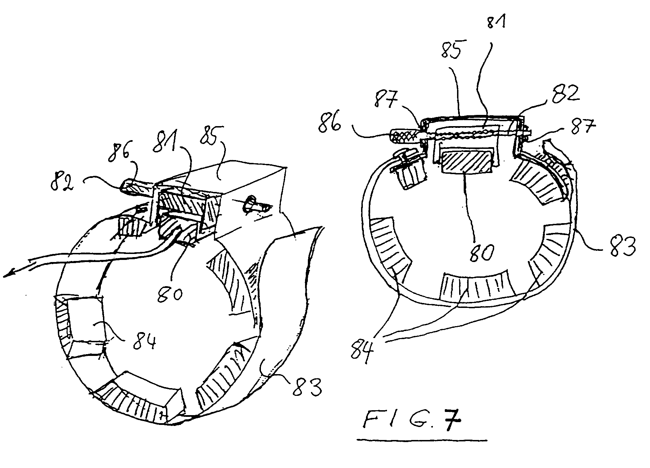

Figur 7 zeigt einen Impulswellenformsensor 40 zusammen mit seiner Montageeinrichtung. Der Sensor 40 ist ein Doppler-Ultraschall-Arterienblutstromsensor und umfaßt einen Doppler-Ultraschallwandler 80, der als flache Packung ausgebildet ist. Hierdurch wird eine stabile und kompakte Anbringung am Arm des Patienten erreicht. Die Doppler-Kristalle sind so montiert, daß sie einen festen Neigungswinkel besitzen, der typischerweise 30° zur Horizontalen beträgt.

Der Wandler wird durch einen Wandlerträger 81 gehalten, der verstellbar an einem Arm 85 gelagert ist. Die beiden Schenkel des Armes dienen zur Befestigung eines Streifens 83, der um den Arm eines Patienten gelegt wird. Der Streifen 83 kann eng anliegend über eine klebemittelfreie Verbindung seiner Enden um den Arm herum befestigt werden, beispielsweise mit Hilfe von Klettmaterial. An seiner Innenseite besitzt der Streifen eine Vielzahl von Stücken 84 aus einem zusammendrückbaren Material, die zur Aufnahme von Stößen und Bewegungen dienen.

Nach der anfänglichen Befestigung des Wandlers in angenäherter Weise wird eine Feineinstellung der Position des Wandlers über eine Einstelleinrichtung vorgenommen, die eine Schraubspindel 82 aufweist, welche sich durch entsprechende Bohrungen im Arm 85 und dem Wandlerträger 81 und durch zwei Halteringe 87 auf beiden Seiten des Armes erstreckt. Die Schraubspindel kann über einen Knopf 86 an ihrem einen Ende manuell betätigt werden. Durch Drehen des Knopfes 86 werden der Träger 81 und dann der Wandler 80 in Querrichtung zum Arm des Patienten bewegt.

Diese Ausführungsform ermöglicht eine zuverlässige Befestigung am Arm ohne Kleber und Aufrechterhaltung eines angemessenen Drucks des Wandlers gegen die gewünschte Stelle auf der Haut.

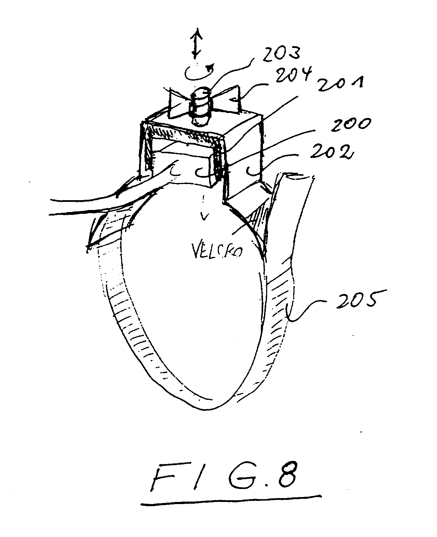

Eine weitere Ausführungsform der Montageeinrichtung für den Wandler ist in Figur 8 dargestellt. Der Wandler 200 ist identisch ausgebildet wie in Figur 7. Er wird ebenfalls von einem Wandlerträger 201 gehaltert, der die Form eines umgedrehten U besitzt. Gemäß dieser Ausführungsform kann der Träger vertikal in der Zeichnung bewegt werden, so daß der Druck, mit dem der Wandler gegen den Arm gepreßt wird, eingestellt werden kann. Dies wird mit Hilfe einer Stellschraube 203 erreicht, die (bei 204) manuell gedreht werden kann und sich durch eine Schraubenbohrung in einem Arm 202 erstreckt. Durch Drehen der Schraube wird daher der Abstand zwischen dem Träger 201 und dem Arm 202 verändert und somit die Wandlerpackung gegen den Arm gepreßt.

Wie bei der Ausführungsform der Figur 7 dienen zwei Schenkel des Armes 202 zur Befestigung eines Streifens 205, der um den Arm eines Patienten gelegt werden kann. Der Streifen kann mit Hilfe einer entsprechenden Verbindung wie in Figur 7 gezeigt eng anliegend befestigt werden.

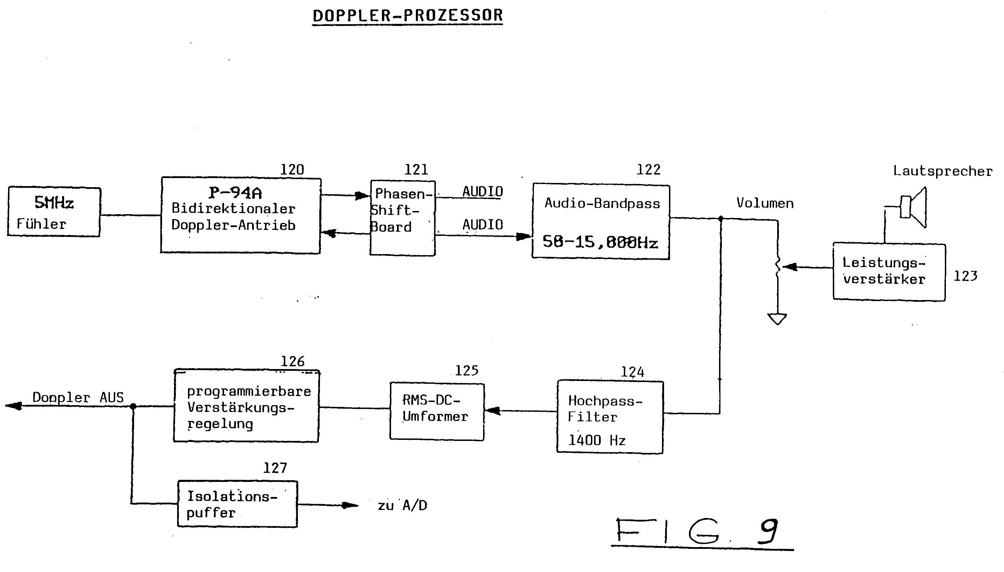

Es wird nunmehr auf eine Ausführungsform eines Impulswellenformprozessors 42 Bezug genommen, von dem ein Blockdiagramm in Figur 9 gezeigt ist. Dieser Prozessor besitzt die folgenden Komponenten:

120 BIDIREKTIONALER DOPPLER-FÜHLER, Modell MEDASONICS P 94-A. Hierbei handelt es sich um einen 5 MHz Doppler-Blutstrommeßwandler, der an die Betreiberschaltung angeschlossen ist.

121 PHASENVERSCHIEBUNGSSCHALTUNG, MEDASONICS p.n. 109-0051-010, trennt die Töne des herannahenden Blutstromes und liefert zwei hochpegelige Audiosignale.

122 AUDIOBANDPASS läßt die Frequenzen zwischen 70 Hz und 15000 Hz durch und unterdrückt Rauschen, insbesondere 50/60 Hz "hum".

123 LEISTUNGSVERSTÄRKER sorgt für den Betrieb des Lautsprechers und die Volumensteuerung von der Frontplatte.

124 HOCHPASSFILTER sondert die Hochfrequenzen vom Audiosignal ab. Der Durchbruch des Blutes erzeugt hochfrequente Signale (über 1400 Hz). Dieses Filter dämpft auch den vom zurückweichenden Strom erzeugten Schall, der niedrigere Frequenzen besitzt.

125 RMS zu DC UMFORMER mißt die Energie des Hochfrequenzspektrums durch Umwandlung des gesamten quadratischen Mittelwertes in eine proportionale Gleichspannung.

126 PROGRAMMIERBARER VERSTÄRKUNGSREGLER ermöglicht eine Verstärkung des RMS-Wertes unter Computerregelung. 3 bits setzen 8 Verstärkungspegel. Das verarbeitete Doppler-Signal steht am BNC-Ausgangsanschluß zur Verfügung.

127 ISOLATIONSPUFFER überführt das verarbeitete Doppler-Signal zum A/D, der gemäß den Sicherheitsnormen für den Patienten isoliert ist.

Bei dieser Ausführungsform stellt der Prozessor ein Analogausgangssignal zur Verfügung, das vorzugsweise proportional zum gesamten raschen Blutstrom ist, d.h. dem Teil des Blutstromes, der vom Sensor 40 erfaßt wird und mit beträchtlicher Geschwindigkeit fließt. Der Prozessor erzeugt ein Ausgangssignal für einen A/D-Wandler, das proportional ist zum quadratischen Mittelwert (RMS) der Amplitude der Doppler/Audioshiftfrequenzen über einer Frequenz von 300 Hz oder einer Frequenz, die dem vielfachen aus der Doppler-Trägerfrequenz und dem Faktor $6 \times 10^{-5}$ entspricht.

Figur 10 zeigt eine exakte Schaltung des Prozessors gemäß Figur 9.

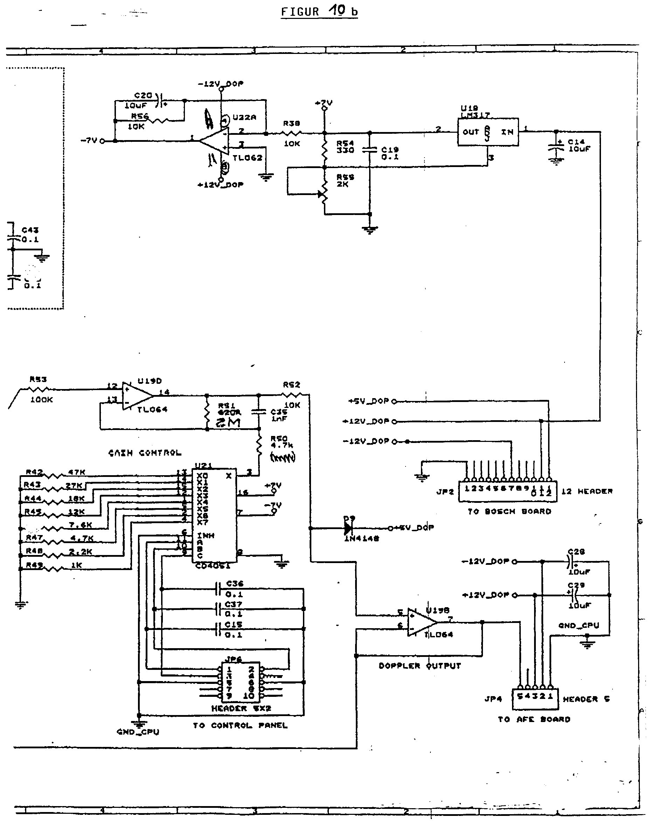

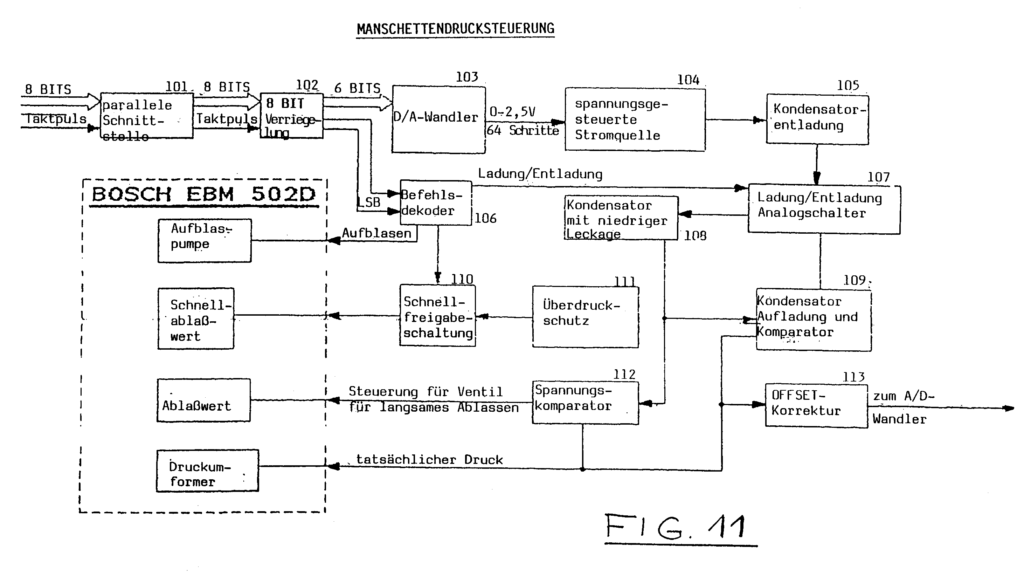

Figur 11 ist ein Blockdiagramm einer Manschettendrucksteuereinheit, d.h. der Pumpensteuereinheit 36, die in Figur 1 gezeigt ist. Eine exakte Schaltung dieser Einheit ist in Figur 12 dargestellt.

Die Manschettendrucksteuereinheit besitzt die folgenden Komponenten:

101 PARALELLE SCHNITTSTELLE ausgebildet als 8-bit Parallelanschluß, D-15 Anschluß, empfängt die Befehle vom PC (Dell Computer). Die zur Verfügung stehenden Befehle sind:

- Aufblasen

- Stop
- langsames Ablassen mit einer vorgegebenen Geschwindigkeit
- schnelles Ablassen

102 LATCH 8-bit speichert den empfangenen Befehl gesteuert durch STROBE-Impuls.

103 DIGITAL/ANALOG-WANLDER verwendet die sechs signifikantesten bits zur Erzeugung von 64 Spannungsschritten (2,56 V voller Maßstab, 40 mV pro bit).

104 SPANNUNGSGEREGELTE STROMQUELLE wandelt die Konstantspannung in konstanten Strom um gemäß:

Strom = Eingangsspannung/20 kohm

was 2 Mikroampere pro bit (126 Mikroampere max) bedeutet.

105 KONDENSATORENTLADUNG ist eine Schaltung, die in der Lage ist, einen 1000 µf Kondensator mit vom Block 104 zur Verfügung gestelltem Konstantstrom in fließender Weise (keine der Klemmen ist an Erde gelegt) zu entladen. Aufgrund der Konstantstromentladung fällt die Spannung über dem Kondensator mit einer konstanten Geschwindigkeit, wiedergegeben durch:

$$dv = 1/c \cdot Zeit \cdot Strom$$

was zu einem Minimalwert von 2 mv/sec und einem Maximalwert von 126 mv/sec führt.

106 BEFEHLSDEKODER empfängt die beiden am wenigstens signifikanten bits des empfangenen Byte und dekodiert die vier Basisbefehle: Aufblasen, Stop, schnelles Ablassen und Ablassen mit einer vorgegebenen Geschwindigkeit.

107 LADE/ENTLADE-SCHALTER schließt den Kondensator mit niedriger Leckage (als Sample & Hold verwendet) an die Lade- oder Entladeschaltung an. Der Analogschalter ist vom DPDT-Typ.

108 KONDENSATOR MIT NIEDRIGER LECKAGE 1000 µf wird als Spannungsspeicher eingesetzt. Die Spannung über den Kondensator folgt dem tatsächlichen Manschettendruckwert. Durch Entladung desselben mit einem konstanten Strom wird eine linear abnehmende Spannung erzeugt.

109 KONDENSATORAUFLADEEINHEIT & KOMPARATOR legt die Spannung über den Kondensator so fest, daß diese dem tatsächlichen Manschettendruckwert folgt. Der Wert wird von der Bosch-Einheit als 1V pro 100 mm Hg Druck empfangen.

110 SCHNELLFREIGABESCHALTUNG ist ein Antrieb für das Schnellfreigabeventil der Bosch-Einheit. Ein rasches Ablassen tritt auf nach dem Empfang des entsprechenden Befehles oder wenn der Druck den maximal erlaubten Wert (300 mm Hg) erreicht.

111 ÜBERDRUCKSCHUTZ ist eine Notschaltung, die die Manschette bei einem Druck von 300 mm Hg vollständig entleert. Dieser Herstellerwert kann durch Verwendung eines internen Potentiometers verändert werden.

112 SPANNUNGSKOMPARATOR stellt die Rückkopplungsschleife dar, die das Ablaßventil von Bosch regelt. Während des langsamen Ablassens wird der Kondensator mit einem programmierten Konstantstrom entladen. Die Spannung über den Kondensator fällt linear ab. Der Komparator vergleicht diese Spannung mit dem tatsächlichen Druckwert. Der verstärkte Fehlerwert treibt das Ablaßventil an. Dies hat zur Folge, daß der Druckk mit dem programmierten Wert abnimmt.

113 AUSGLEICHSKORREKTUR ermöglicht die Eichung des Analogdruckwertes gegenüber einem Standardmanometer.

Die Vorrichtung besitzt das folgende Funktionsprinzip:

Nach Empfang (über den Paralleleingang) des Befehls AUFBLASEN wird die Pumpe eingeschaltet und bläst die Manschette auf, bis der STOP-Befehl empfangen wird. Während des Aufblasens wird der Kondensator genau auf einen Spannungswert aufgeladen, der dem tatsächlichen Druck entspricht.

Der Befehl LANGSAMES ABLASSEN enthält 6 bits, die schließlich in einen Konstantstrom umgewandelt werden. Dieser Strom entlädt den Kondensator, wodurch eine innere eingebaute lineare Spannungsrampe erzeugt wird. Der Komparator vergleicht diese Spannung mit dem Druckwert, wobei die Differenz verstärkt wird. Durch die Fehlerspannung wird das Ablaßventil angetrieben, wodurch bewirkt wird, daß der Druck der Rampe folgt. Mit den beschriebenen Werten beträgt der minimale Ablaßgrad 0,2 mm Hg pro sec, während der maximale Wert 12,6 mm Hg pro sec beträgt.

Durch den Befehl RASCHES ABLASSEN wird die Luft aus der Manschette sofort abgelassen.

Durch den STOP-Befehl wird der Manschettendruck auf dem letzten Wert gehalten.

Im folgenden wird ein Ablaufdiagramm erläutert, das die Funktionsweise der in den Figuren 1 bis 6 dargestellten Vorrichtung beschreibt.

Flußdiagramm der Funktionsweise des Herzmonitors Schritte 10-20: Herrichtung des Patienten und Aufbau der Einrichtung

10

Indiziere Gamma-Tracer, setze Patienten an Gamma-Kamera. Befestige EKG-Elektroden, Blutdruckmonitor, Druckwellenformsensor.

20

Verifiziere visuell akzeptables EKG und Druckwellenform auf Videoanzeige

Verifiziere akzeptable Funktionsweise der Gamma-Kamera. Schritt 30 - Operator fordert Initialisierung der Messung.

Schritte 40-90: Initialisierung der Messung.

40 - Miß MAXAMP, MINAMP, Fortpflanzungszeit für jeden von 10 aufeinanderfolgenden Herzzyklen:

o   miß die maximalen und minimalen Druckwellenformwerte, MAXAMP und MINAMP.

o   Verwende Ankunftssignal zur Errechnung der Fortpflanzungszeit.

50

Erhalte diastolischen und systolischen Druck und Herzschlagmessungen vom MIBP-Monitor.

60

Berechne DP = (systolisch-diastolisch)

70

Berechne Manschettendruckeinstellungen:

P0   = 1,25 · systolisch
P1   = systolisch
P2   = systolisch - 0,25 · DP
P3   = systolisch - 0,50 · DP
P4   = systolisch - 0,65 · DP
P5   = systolisch - 0,75 · DP
P6   = systolisch - 0,85 · DP
P7   = systolisch - 0,90 · DP
P8   = systolisch - 0,95 · DP
P9   = diastolisch.

Schritte 80-90 (während Initialisierung) miß Basislinien-Impulswellenformwerte bei keinem Blutstrom.

80

Blase Manschette 38 auf P0 auf.

90

Wiederhole.

Berechne kumulative durchschnittliche Druckwellenformamplitude AMP für 10 sec

Rücksetze Manschettendruck-Increment-Flag.

Wenn (AMP ≥ 0,05 (MAXAMP - MINAMP))

o   erhöhe Manschettendruck um 10%
o   setze Manschettendruck-Increment-Flag

bis Manschettendruck-Increment-Flag nicht gesetzt.

Schritte 100-260: CPI-Messung

Schritte 100-170: Messung des Blutdrucks als Funktion der Zeit.

100

Für i = 1 bis 8

Setze Manschettendruck auf nächsten Wert Pi

110

für j = 1 bis N (N ist typischerweise 5-10)

112

Nachdem der neue Druck erreicht ist, erfasse den nächsten QRS-Komplex

114

Wende Ankunft des Verfahrens an, um Durchbruchzeit herauszufinden, speichere im Element Druckzeit (i, j) des Schemas Druckzeit.

Nächstes j

120

Ersetze die außerhalb liegenden Druck-Zeit-Punkte durch gebündelte Werte, berechne durchschnittliche Zeit für jeden Druck und speichere im Feld Druckpunkte.

Wiederhole

122

Berechne durchschnittliche TIMEAVE und Standardabweichung TDEV der Elemente Druck-Zeit (i, k), k = 1 bis N.

124

Rücksetze ersetzten Punkt.

126

Finde den ersten Druck-Zeit-Wert (i, k), k = 1 bis N, der außerhalb des Bereiches [TIMEAVE +/- 3 TDEV] liegt. Wenn ein solcher Punkt gefunden wurde:

o   Streiche ihn aus dem Druck-Zeit-Feld.

o   Ersetze ihn durch Durchführung einer neuen Messung (Schritte 112, 114).

o   Setze Flag für ausgetauschten Punkt.

Bis Flag für ausgetauschten Punkt nicht gesetzt ist: Fülle die Druckpunkte des Feldes mit den um die Fortpflanzungszeit verschobenen Ankunftszeiten und den entsprechenden Manschettendrücken aus.

Druckpunkte (i, 1) = Pi:

Druckpunkte (i, 2) = TIMAVE - Fortpflanzungszeit

Nächstes i (Manschettendruckeinstellung).

Druckpunkte (9, 1) = P9 (d.h. diastolischer Wert)

Druckpunkte (9, 2) = 0

160

Interpoliere die Werte des Feldes Druckpunkte unter Verwendung einer stückweisen, polynomischen, durch die Methode der kleinsten Quadrate ermittelten Ausgleichskurve auf die Druck-Zeit-Paare, um nach dem Beginn der Systole einen Druckwert für jede Millisekunde zu erhalten, und speichere jeden Wert im Feld der Druckkurve.

180
Erhalte die volumetrischen Stichprobenwerte und die Nach-QRS-Meßzeiten von der Gamma-Kamera über deren CPU und speichere diese Werte im Feld Volumenpunkte.

190
Interpoliere die Werte des Feldes Volumenpunkte unter Verwendung einer stückweisen, polynomischen Interpolationsfunktion und speichere die Werte für jede Millisekunde nach QRS im Feld Volumenkurve.

200
Beginne mit i = 1 beim Beginn der Systole,
für i = 1 bis K Stichprobenpunkte
(K ist typischerweise die Zahl der Millisekunden während des Anstiegs der Systole)

$$\text{Arbeit (i) = Druckkurve (i)} \cdot \text{Volumenkurve (i)}$$

210
Arbeit (0) = Arbeit (1)
Für i = 1 bis K errechne die Ableitung von Arbeit (i), verwende üblicherweise Mittelwertdifferenzverfahren:

$$\text{Leistung (i) = (Arbeit (i+1) - Arbeit (1-1))/2}$$

220
Ermittle Leistung (i) für maximalen Leistungswert MAXPOWER zum jeweiligen Zeitpunkt, IMAX.

230
Passe mit der Methode der kleinsten Quadrate ermittelte lineare Regressionskurve an alle Punkte der Leistung von i = 1 bis IMAX an; errechne die Standardabweichung der Daten aus der gezogenen Linie.

240
Für 1 bis K streiche sämtliche Eingänge aus Leistung (i), die Werte außerhalb von drei Standardabweichungen von der gezogenen Linie besitzen. Wenn die Punkte gestrichen sind, kehre zu 220 zurück.

250
Passe lineare Regressionskurve an alle Punkte an.

260
Der endgültige CPI-Wert ist die Steigung der linearen Regressionskurve.

Verfahren Ankunft: Miß Fortpflanzungszeit

Mit A/D-Wandler 44 sammle mindestens 1000 Proben pro Sekunde

1    Erfasse die Zeit TQRS der R-Wellenauslösung
2    Beginne Taktzählung
3    Warte 50 msec
4    von den folgenden 30 msec Proben berechne das Mittel LOWMEAN und die Standardabweichung LOWSD und ziehe eine mit der Methode der kleinsten Quadrate ermittelte lineare Regressionskurve LOWLINE an die Proben
5    Erfasse die ersten 30 folgenden Punkte, deren Werte alle größer sind als (LOWMEAN + 3 LOWSD)
6    Passe eine mit der Methode der kleinsten Quadrate ermittelte Regressionslinie HIGHLINE an die 30 msec der Punkte
7    Berechne den Start der Systole TSYS1 als den Zeitpunkt, an dem sich LOWLINE und HIGHLINE schneiden
8    Berechne die Zahl der msec von TQRS bis TSYS1.

**Patentansprüche**

1.    Verfahren zum nichtinvasiven Messen des Herzleistungsindex als Maß für die Herzfunktion unter Ruhe- und Bewegungsbedingungen mit den folgenden Schritten:

nichtinvasives Messen des Drucks der linken Herzkammer in Abhängigkeit von der Zeit durch Messen der Ankunftszeiten von Herzdruckimpulsen an einer vorgegebenen arteriellen Stelle im Abstand vom Herz bei einer Vielzahl von Manschettendruckwerten;

nichtinvasives Messen des Volumens der linken Herzkammer in Abhängigkeit von der Zeit;

Bestimmen der Arbeit der linken Herzkammer als Produkt aus dem Druck der linken Herzkammer und dem Volumen der linken Herzkammer in Abhängigkeit von der Zeit;

Bestimmen der Leistung der linken Herzkammer als Ableitung dieses Produktes nach der Zeit; und

Bestimmen der Steigung dieser zeitlichen Ableitung in der Anstiegsphase während des Intervalls vom Beginn der Systole bis zum Augenblick maximaler Leistung als Wert für den Herzleistungsindex.

2.    Verfahren nach Anspruch 1, dadurch gekennzeichnet, daß der Schritt des Messens des Drucks der linken Herzkammer das Konzentrieren der größten Zahl der Druckmessungen in dem Intervall während der frühen Ausstoßphase der linken Herzkammer umfaßt.

3.    Verfahren nach Anspruch 1 oder 2, dadurch gekennzeichnet, daß der Schritt des Messens des Drucks der linken Herzkammer ferner den Schritt des Messens der Ankunftszeiten von Herzdruckimpulsen an der vorgegebenen Stelle während der Zeitdauer umfaßt, während der der Druck der linken Herzkammer von 100 % auf 125 % des enddiastolischen Wertes ansteigt.

4. Verfahren nach einem der vorangehenden Ansprüche, dadurch gekennzeichnet, daß es den Schritt der Anzeige eines Realzeit-Elektrokardiogrammes und von Blutdruckwellen auf einer kontinuierlich aktualisierten Basis umfaßt.

5. Verfahren nach Asnpruch 4, dadurch gekennzeichnet, daß es die Anzeige der Druckwerte der linken Herzkammer und der entsprechenden volumetrischen Werte der linken Herzkammer gleichzeitig und zusammen mit dem Elektrokardiogramm und den Blutdruckwellen umfaßt.

6. Verfahren nach einem der vorangehenden Ansprüche, dadurch gekennzeichnet, daß es den Schritt des Messens der Ankunftszeit für einen vorgegebenen Sperrdruck während eines oder mehreren Herzzyklen und der Speicherung der gemessenen Zeiten für jeden Druck umfaßt.

7. Verfahren anch einem der vorangehenden Ansprüche, dadurch gekennzeichnet, daß der Schritt des Messens der Ankunftszeit den Schritt der Zurückweisung von Zeitwerten umfaßt, die eine nicht akzeptierbare Varianz besitzen.

8. Verfahren nach einem der vorangehenden Ansprüche, dadurch gekennzeichnet, daß der Schritt des Messens der Ankunftszeit ferner den Schritt der statistischen Durchschnittsbildung von diversen annehmbaren Stichprobenpunkten umfaßt, um auf diese Weise Effekte der Varianz von Schlag zu Schlag, von Störsignalen und des Verrauschens herabzusetzen.

9. Verfahren nach einem der vorangehenden Ansprüche, dadurch gekennzeichnet, daß der Schritt des Messens des Volumens der linken Herzkammer die Durchführung von mindestens einer Messung innerhalb von 15 msec des QRS-Komplexes umfaßt.

10. Verfahren nach einem der vorangehenden Ansprüche, dadurch gekennzeichnet, daß der Schritt der Messung des Volumens der linken Herzkammer die Durchführung von mehreren Volumenmessungen innerhalb von 40 msec umfaßt.

11. Verfahren nach einem der vorangehenden Ansprüche, dadurch gekennzeichnet, daß der systolische und diastolische Blutdruck gemessen wird.

12. Verfahren nach einem der vorangehenden Ansprüche, dadurch gekennzeichnet, daß der Herzleistungsindex als Steigung der nach der Methode der kleinsten Quadrate ermittelten Regressionsanpassungskurve an eine Reihe von momentanen Leistungswerten bis zu einem maximalen Leistungspunkt errechnet wird, wobei Punkte ausgeschlossen werden, deren Werte außerhalb des Varianzbereiches liegen, der den anderen Punkten entspricht.

13. Verfahren nach einem der vorangehenden Ansprüche, dadurch gekennzeichnet, daß in Anpassung an die Ankunftszeiten bei der Vielzahl der Druckwerte eine Kurve gezogen wird, die sich der zeitabhängigen Welle des Drucks der linken Herzkammer annähert.

14. Verfahren nach einem der vorangehenden Ansprüche, dadurch gekennzeichnet, daß die Ankunftszeiten der Herzdruckimpulse gemessen werden, indem die Doppler-Signale des Blutstromes an der vorgegebenen Stelle gemessen werden.

15. Verfahren nach einem der vorangehenden Ansprüche, dadurch gekennzeichnt, daß die Ankunftszeiten der Herzdruckimpulse unter Bewegungs-Stress-Bedingungen gemessen werden.

16. Vorrichtung zum Durchführen des Verfahrens nach einem der Ansprüche 1 bis 15 mit

Einrichtungen (30-44) zum nichtinvasiven Messen des Drucks der linken Herzkammer in Abhängigkeit von der Zeit durch Messen der Ankunftszeiten von Herzdruckimpulsen an einer vorgegebenen arteriellen Stelle im Abstand vom Herz bei einer Vielzahl von Manschettendruckwerten;

Einrichtungen (60, 62) zum nichtinvasiven Messen des Volumens der linken Herzkammer in Abhängigkeit von der Zeit;

Einrichtungen (20) zum Bestimmen der Arbeit der linken Herzkammer als Produkt aus dem Druck der linken Herzkammer und dem Volumen der linken Herzkammer in Abhängigkeit von der Zeit;

Einrichtungen (20) zum Bestimmen der Leistung der linken Herzkammer als Ableitung dieses Produktes nach der Zeit; und

Einrichtungen (20) zum Bestimmen der Steigung dieser zeitlichen Ableitung in der Anstiegsphase während des Intervalls vom Beginn der Systole bis zum Augenblick maximaler Leistung als Wert für den Herzleistungsindex.

17. Vorrichtung nach Anspruch 16, dadurch gekennzeichnet, daß die Einrichtungen (30-44) zum Messen des Drucks der linken Herzkammer ferner Einrichtungen zum Konzentrieren der größten Zahl der Druckmessungen in dem Intervall während der frühen Ausstoßphase umfassen.

18. Vorrichtung nach Anspruch 16 oder 17, dadurch gekennzeichnet, daß die Einrichtungen (30-44) zum Messen des Drucks der linken Herzkammer ferner Einrichtungen zum Messen der Ankunftszeiten der Herzdruckimpulse an der vorgegebenen Stelle während einer Zeitdauer umfassen, während der der Druck der linken Herzkammer von 100 % bis auf 125 % des enddiastolischen Wertes ansteigt.

19. Vorrichtung nach einem der Ansprüche 16 bis 18, dadurch gekennzeichnet, daß sie ferner Einrichtungen zum Anzeigen eines Realzeit-Elektrokardiogrammes und von Blutdruckwellen auf einer kontinuierlich aktualisierten Basis umfaßt.

20. Vorrichtung nach Anspruch 19, dadurch gekennzeichnet, daß sie Einrichtungen zur Anzeige der Druckwerte der linken Herzkammer und der errechneten entsprechenden volumetrischen Werte der linken Herzkammer gleichzeitig und zusammen mit dem Elektrokardiogramm und den Blutdruckwellen aufweist.

21. Vorrichtung nach einem der Ansprüche 16 bis 20, dadurch gekennzeichnet, daß sie Einrichtungen zum Messen der Ankunftszeit für einen vorgegebenen Absperrdruck während eines oder mehreren Herzzyklen und zur Speicherung der gemessenen Zeiten für jeden Druck umfaßt.

22. Vorrichtung nach einem der Ansprüche 16 bis 21, dadurch gekennzeichnet, daß die Einrichtungen zum Messen der Ankunftszeit Einrichtungen zum Zurückweisen von Zeitwerten, die eine nicht akzeptierte Varianz besitzen, umfassen.

23. Vorrichtung nach einem der Ansprüche 16 bis 22, dadurch gekennzeichnet, daß die Einrichtungen zum Messen der Ankunftszeit auch Einrichtungen zur statistischen Durchschnittsbildung von diversen akzeptablen Stichprobenpunkten umfassen, um die Auswirkungen der Varianz von Schlag zu Schlag, von Störsignalen und des Verrauschens herabzusetzen.

24. Vorrichtung nach einem der Ansprüche 16 bis 23, dadurch gekennzeichnet, daß die Einrichtungen (60, 62) zum Messen des Volumens der linken Herzkammer Einrichtungen zur Durchführung von mindestens einer Messung innerhalb von 15 msec des QRS-Komplexes aufweisen.

25. Vorrichtung nach Anspruch 24, dadurch gekennzeichnet, daß die Einrichtungen (60, 62) zum Messen des Volumens der linken Herzkammer Einrichtungen zur Durchführung einer Vielzahl von Volumenmessungen innerhalb von 40 msec umfassen.

26. Vorrichtung nach einem der Ansprüche 16 bis 25, dadurch gekennzeichnet, daß sie Einrichtungen zum Messen des systolischen und diastolischen Blutdrucks umfaßt.

27. Vorrichtung nach einem der Ansprüche 16 bis 26, dadurch gekennzeichnet, daß sie Einrichtungen (20) zum Berechnen des Herzleistungsindex als Steigung der nach der Methode der kleinsten Quadrate ermittelten Regressionsanpassungskurve an die gesamte Reihe von momentanen Leistungswerten bis zu einem maximalen Leistungspunkt mit Ausnahme von Punkten, deren Werte außerhalb des Varianzbereiches liegen, der den anderen Punkten entspricht, umfaßt.

28. Vorrichtung nach einem der Ansprüche 16 bis 27, dadurch gekennzeichnet, daß sie ferner einen Impulswellensensor (40) und einen Impulswellenprozessor (42) aufweist, die so arbeiten, daß Störeinflüsse durch Bewegungen ausgeschaltet werden.

29. Vorrichtung nach Anspruch 28, dadurch gekennzeichnet, daß die Einrichtungen zum Erfassen der Ankunft der Herzdruckwelle an einer vorgegebenen arteriellen Stelle, vorzugsweise einer Stelle der Armarterie, durch einen Doppler-Ultraschall-Arterienwandbewegungssensor gebildet sind.

30. Vorrichtung nach Anspruch 28, dadurch gekennzeichnet, daß die Einrichtungen zum Erfassen der Ankunft der Herzdruckwelle an einer vorgegebenen arteriellen Stelle, vorzugsweise einer Stelle der Armarterie, durch einen Doppler-Ultraschall-Blutstromsensor gebildet sind.

31. Vorrichtung nach einem der Ansprüche 16 bis 30, dadurch gekennzeichnet, daß sie Einrichtungen zum Ausschalten von Bewegungsstöreinflüssen aufweist, die einen Doppler-Sensorhalter und Einrichtungen zum Zurückweisen von niedrigen Frequenzen aus dem Doppler-Audioshiftspektrum umfassen.

32. Vorrichtung nach Anspruch 31, dadurch gekennzeichnet, daß der Doppler-Ultraschallsensor (Wandler) (80, 200) von einem Armband gehalten wird, das einen verstellbaren Wandlerträger (81, 201) aufweist, der an einem verstellbaren Befestigungsstreifen (83, 205) befestigt ist.

33. Vorrichtung nach einem der Ansprüche 30 bis 32, dadurch gekennzeichnet, daß der Doppler-Ultraschallsensor (Wandler) (80, 200) als flache Packung mit Doppler-Kristallen ausgebildet ist, die einen festen Neigungswinkel zur Horizontalen besitzen, der typischerweise 30° beträgt.

34. Vorrichtung nach Anspruch 28, dadurch gekennzeichnet, daß der Impulswellenprozessor (42) ein Hochpassfilter (124) aufweist, das die Hochfrequenzen vom Audio-Signal abtrennt, sowie einen RMS-Amplituden-Gleichstromumformer (125), der die Energie des Hochfrequenzspektrums mißt und den gesamten RMS (quadratischen Mittelwert) in eine proportionale Gleichspannung umwandelt.

**Claims**

1. A method for noninvasively measuring the value of cardiac power index as a descriptor of performance of a heart under conditions of rest and movement comprising the following steps:

    noninvasively measuring the left ventricular pressure of the heart with reference to time by measuring the arrival times of cardiac pressure pulses at a given arterial site spaced from the heart at a plurality of cuff pressure values;

    noninvasively measuring the left ventricular volume of the heart with reference to time;

    determining the work performed by the left ventricle as the product of the left ventricular pressure and the left ventricular volume as a function of time;

    determining the power of the left ventricle as the time derivative of said product; and

    determining the slope of this time derivative in the rising phase during the interval from the onset of systole to the moment of maximum power, thereby to provide the value of the cardiac power index.

2. The method according to claim 1, characterized in that the step of measuring the left ventricular pressure comprises the step of concentrating the largest number of pressure measurements in the interval during the early ejection phase of the left ventricle.

3. The method according to claim 1 or 2, characterized in that the step of measuring the left ventricular pressure also comprises the step of measuring the arrival times of cardiac pressure pulses at the given site during the time period during which the left ventricular pressure rises from 100 % to 125 % of the end-diastolic value.

4. The method according to one of the preceding claims, characterized in that it also comprises the step of displaying a real-time electrocardiogram and blood pressure wave forms on a continuously updated basis.

5. The method according to claim 4, characterized in that it comprises the steps of displaying simultaneously and together with said electrocardiogram and pressure wave forms, the left ventricle pressure and corresponding left ventricular volumetric values.

6. The method according to one of the preceding claims, characterized in that it comprises the step of measuring, during one or more cardiac cycles, the arrival time for a predetermined occlusive pressure, and storing the measured times for each pressure.

7. The method according to one of the preceding claims, characterized in that the step of measuring the time of arrival includes the step of rejecting time values having unacceptable variance.

8. The method according to one of the preceding claims, characterized in that the step of measuring the arrival time also includes the step of statistical averaging of several acceptable time measurements to reduce the effects of beat-to-beat variance, artifactual signals and noise.

9. The method according to one of the preceding claims, characterized in that the step of measuring the left ventricular volume includes the step of taking at least one measurement within 15 msec of the QRS-complex.

10. The method according to one of the preceding claims, characterized in that the step of measuring the left ventricular volume includes the step of carrying out multiple volume measurements within 40 msec.

11. The method according to one of the preceding claims, characterized by measuring the systolic and diastolic blood pressure.

12. The method according to one of the preceding claims, characterized by calculating the cardiac power index as the slope of the best least squares regression fit to an entire set of instantaneous power values up to a maximum power point, excluding points whose values lie outside the range of variance that is commensurate with the other points.

13. The method according to one of the preceding claims, characterized by drawing a graph in adaption to the arrival times at the plurality of pressure values, said draft approaching the time-dependent wave of the pressure of the left ventricle.

14. The method according to one of the preceding claims, characterized by measuring the arrival times of the cardiac pressure pulses by measuring the Doppler signals of the blood stream at the predetermined site.

15. The method according to one of the preceding claims, characterized by measuring the arrival times of the cardiac pressure pulses under conditions of movement-stress.

16. An apparatus for carrying out the method according to one of the claims 1 to 15 comprising:

   means (30-44) for noninvasively measuring the left ventricular pressure of the heart with respect to time by measuring the arrival times of cardiac pressure pulses at a predetermined arterial site spaced from the heart at a plurality of caft pressure values;

   means (60, 62) for noninvasively measuring the left ventricular volume of the heart with respect to time;

   means (20) for determining the work of the left ventricle as the product of the left ventricular pressure and the left ventricular volume as function of time;

   means (20) for determining the power of the left ventricle as the time derivative of said product; and

   means (20) for determining the slope of the time derivative in the rising phase during the interval from the onset of systole to the time of maximum power as value for the cardiac power index.

17. The apparatus according to claim 16, characterized in that the means (30-44) for measuring the left ventricular pressure also comprises means for concentrating the largest number of pressure measurements in the interval during the early ejection phase.

18. The apparatus according to claim 16 or 17, characterized in that the means (30-44) for measuring the left ventricular pressure also comprises means for measuring the arrival times of cardiac pressure pulses at the predetermined site during the time period during which the left ventricular pressure rises from 100 % to 125 % of the end-diastolic value.

19. The apparatus according to one of the claims 16 to 18, characterized in that it further includes means for displaying an real-time electrocardiogram and blood pressure wave forms on a continuously updated basis.

20. The apparatus according to claim 19, characterized in that it includes means for displaying, simultaneously and together with said electrocardiogram and pressure wave forms, left ventricular pressure values and the calculated corresponding left ventricular volumetric values.

21. The apparatus according to one of the claims 16 to 20, characterized in that it includes means for measuring, during at least one cardiac cycle or a plurality of cardiac cycles, the arrival time for a predetermined occlusive pressure, and for storage of the measured times for each pressure.

22. The apparatus according to one of the claims 16 to 21, characterized in that the means measuring the time of arrival includes means for rejecting time values having unacceptable variance.

23. The apparatus according to one of the claims 16 to 22, characterized in that the means for measuring the time of arrival also includes means for statistical averaging of several acceptable time measurements to reduce the effects of beat-to-beat variance, artifactual signals and noise.

24. The apparatus according to one of the claims 16 to 23, characterized in that the means (60, 62) for measuring the left ventricular volume includes means for taking at least one measurement within 15 msec of the QRS-complex.

25. The apparatus according to claim 24, characterized in that the means (60, 62) for measuring the left ventricular volume includes means for carrying out a plurality of volume measurements within 40 msec.

26. The apparatus according to one of the claims 16 to 25, characterized in that it includes means for measuring the systolic and diastolic blood pressure.

27. The apparatus according to one of the claims 16 to 26, characterized in that it comprises means (20) for calculating the cardiac power index as the slope of the best least squares regression fit to an entire set of instantaneous power values up to a maximum power point, excluding points whose variance is not commensurate with the other points.

28. The apparatus according to one of the claims 16 to 27, characterized in that it further includes a pulse wave sensor (40) and a pulse wave processor (42) operative to reject motion artifact.

29. The apparatus according to claim 28, characterized in that the means for detecting the arrival of the cardiac pressure wave at a predetermined arterial site, preferably a site of the artery of the arm, is a Doppler ultrasound arterial wall motion sensor.

30. The apparatus according to claim 28, characterized in that the means for detecting the arrival of the cardiac pressure wave at a given arterial site, preferably a site of the artery of the arm, is a Doppler ultrasound blood flow sensor.

31. The apparatus according to one of the claims 16 to 30, characterized in that it contains means for excluding motion artifact, said means comprising a Doppler sensor holder and means for rejecting low frequencies from the Doppler audio shift spectrum.

32. The apparatus according to claim 31, characterized in that the Doppler ultrasound sensor (transducer) (80, 200) is held by an armband comprising an adjustable transducer carrier (81, 201) fixed to an adjustable attachment strap (83, 205).

33. The apparatus according to one of the claims 30 to 32, characterized in that the Doppler ultrasound sensor (transducer) (80, 200) is formed as flat package with Doppler crystals having a fixed angle of inclination with respect to horizontal which is typically 30°.

34. The apparatus according to claim 28, characterized in that the pulse wave processor (42) includes a high-pass filter (124) separating the high frequencies from the audio signal and a RMS-amplitude-to-DC-converter (125) measuring the power of the high frequency spectrum and converting the total RMS (quadratic mean value) into a proportional DC voltage.

**Revendications**

1. Procédé pour la mesure non invasive de l'indice de puissance du coeur, comme mesure du fonctionnement du coeur dans des conditions de repos et de mouvement, comportant les étapes qui consistent à:

mesurer de manière non invasive l'évolution de la pression dans le ventricule gauche en fonction du temps, en mesurant l'instant d'arrivée des impulsions de pression cardiaque à un emplacement prédéterminé d'une artère à distance du coeur, à de nombreuses valeurs de la pression du manchon;
mesurer de manière non invasive l'évolution du volume du ventricule gauche en fonction du temps;
déterminer le travail du ventricule gauche, comme produit de la pression dans le ventricule gauche et du volume du ventricule gauche, en fonction du temps;
déterminer l'évolution la puissance du ventricule gauche, par dérivation de ce produit par rapport au temps; et
déterminer la pente de cette dérivée par rapport au temps pendant la phase de montée, au cours de l'intervalle qui va du début de la systole jusqu'au moment où l'on obtient la puissance maximale, comme valeur de l'indice de puissance du coeur.

2. Procédé selon la revendication 1, caractérisé en ce que l'étape consistant à mesurer la pression dans le ventricule gauche comporte la concentration du plus grand nombre des mesures de pression dans l'intervalle correspondant à la phase de contraction précoce du ventricule gauche.

3. Procédé selon les revendications 1 ou 2, caractérisé en ce que l'étape consistant à mesurer la pression dans le ventricule gauche comporte en outre l'étape consistant à mesurer les instants d'arrivée des impulsions de pression cardiaque à l'emplacement prédéterminé, pendant la période au cours de laquelle la pression dans le ventricule gauche monte de 100% à 125% de sa valeur en fin de diastole.

4. Procédé selon l'une des revendications précédentes, caractérisé en ce qu'il comporte l'étape consistant à afficher un électrocardiogramme en temps réel et les ondes de pression sanguine sur base actualisée en continu.

5. Procédé selon la revendication 4, caractérisé en ce qu'il comporte l'affichage des valeurs de pression dans le ventricule gauche et des valeurs correspondantes du volume du ventricule gauche, simultanément et en même temps que l'affichage de l'électrocardiogramme et celui des ondes de pression sanguine.

6. Procédé selon l'une des revendications précédentes, caractérisé en ce qu'il comporte l'étape consistant à mesurer l'instant d'arrivée pour une pression de blocage prédéterminée pendant un ou plusieurs cycles cardiaques, et la mise en mémoire des instants mesurés pour chaque pression.

7. Procédé selon l'une des revendications précédentes, caractérisé en ce que l'étape consistant à mesurer les instants d'arrivée comporte l'étape consistant à rejeter les valeurs de temps qui présentent une variance non acceptable.

8. Procédé selon l'une des revendications précédentes, caractérisé en ce que l'étape consistant à mesurer les temps d'arrivée comporte en outre l'étape consistant à former la moyenne statistique de différents points de sondage acceptables, pour de cette manière réduire les effets de la variance d'une pulsation à l'autre, les effets de signaux perturbateurs et ceux du bruit.

9. Procédé selon l'une des revendications précédentes, caractérisé en ce que l'étape consistant à mesurer le volume du ventricule gauche comporte l'exécution d'au moins une mesure pendant 15 msec du complexe QRS.

10. Procédé selon l'une des revendications précédentes, caractérisé en ce que l'étape consistant à mesurer le volume du ventricule gauche comporte l'exécution de nombreuses mesures du volume en un laps de temps de 40 msec.

11. Procédé selon l'une des revendications précédentes, caractérisé en ce que l'on mesure la pression sanguine pendant la systole et la diastole.

12. Procédé selon l'une des revendications précédentes, caractérisé en ce que l'on calcule l'indice de puissance du coeur en tant que pente de la courbe d'adaptation par régression, calculée suivant la méthode des moindres carrés, d'une série de valeurs de puissance instantanées jusqu'à un point de puissance maximale, et l'on exclut les points dont les valeurs sont situées à l'extérieur de la plage de variance qui correspond aux autres points.

13. Procédé selon l'une des revendications précédentes, caractérisé en ce que, pour l'adaptation des instants d'arrivée à plusieurs des valeurs de pression, on trace une courbe qui s'approche des ondes temporelles de pression dans le ventricule gauche.

14. Procédé selon l'une des revendications précédentes, caractérisé en ce que l'on mesure les instants d'arrivée des impulsions de pression cardiaque en mesurant les signaux Doppler du flux sanguin à l'emplacement prédéterminé.

15. Procédé selon l'une des revendications précédentes, caractérisé en ce que l'on mesure les instants d'arrivée des impulsions de pression cardiaque dans des conditions de mouvement et de stress.

16. Dispositif en vue de l'exécution du procédé selon l'une des revendications 1 à 15, comportant:

des dispositifs (30-44) pour la mesure non invasive de la pression dans le ventricule gauche en fonction du temps, par mesure des instants d'arrivée d'impulsions de pression cardiaque en un emplacement prédéterminé d'une artère à distance du coeur, pour un grand nombre de valeurs de la pression du manchon;
des dispositifs (60, 62) pour la mesure non invasive de l'évolution du volume du ventricule gauche en fonction du temps;
des dispositifs (20) pour la détermination du travail du ventricule gauche en tant que produit de la pression dans le ventricule gauche et du volume du ventricule gauche par rapport au temps;
des dispositifs (20) pour déterminer la puissance du ventricule gauche en tant que dérivée de ce produit par rapport au temps; et
des dispositifs (20) pour déterminer la pente de cette dérivée par rapport au temps au cours de la phase de montée, pendant l'intervalle qui s'écoule entre le début de la systole et l'instant où la puissance est maximale, comme valeur de l'indice de la puissance cardiaque.

17. Dispositif selon la revendication 16, caractérisé en ce que les dispositifs (30-44) pour la mesure de la pression dans le ventricule gauche comportent en outre des dispositifs pour concentrer le plus grand nombre des mesures de pression dans l'intervalle qui s'écoule pendant la phase de contraction précoce.

18. Dispositif selon les revendications 16 ou 17, caractérisé en ce que les dispositifs (30-44) pour la mesure de la pression dans le ventricule gauche comportent en outre des dispositifs pour la mesure des instants d'arrivée des impulsions de pression cardiaque à l'emplacement prédéterminé, pendant un laps de temps au cours duquel la pression dans le ventricule gauche augmente de 100% à 125% de sa valeur en fin de diastole.

19. Dispositif selon l'une des revendications 16 à 18, caractérisé en ce qu'il comporte en outre des dispositifs pour l'affichage d'un électrocardiogramme en temps réel et des ondes de pression sanguine sur base actualisée en continu.

**20.** Dispositif selon la revendication 19, caractérisé en ce qu'il présente des dispositifs pour l'affichage des valeurs de pression dans le ventricule gauche et des valeurs correspondantes calculées du volume du ventricule gauche, simultanément et en même temps que l'affichage de l'électrocardiogramme et que celui des ondes de pression sanguine.

**21.** Dispositif selon l'une des revendications 16 à 20, caractérisé en ce qu'il comporte des dispositifs pour la mesure des instants d'arrivée à une pression de blocage prédéterminée, pendant un ou plusieurs cycles cardiaques, et pour la mise en mémoire des instants mesurés pour chaque pression.

**22.** Dispositif selon l'une des revendications 16 à 21, caractérisé en ce que les dispositifs pour la mesure des instants d'arrivée comportent des dispositifs pour le rejet des valeurs de temps qui présentent une variance non acceptée.

**23.** Dispositif selon l'une des revendications 16 à 22, caractérisé en ce que les dispositifs pour la mesure des instants d'arrivée comportent également des dispositifs pour la formation d'une moyenne statistique de divers points de sondage acceptables, pour réduire les effets de la variance d'une pulsation à l'autre, les effets de signaux perturbateurs et ceux du bruit.

**24.** Dispositif selon l'une des revendications 16 à 23, caractérisé en ce que les dispositifs (60, 62) pour la mesure du volume du ventricule gauche présentent des dispositifs en vue d'exécuter au moins une mesure pendant 15 msec du complexe QRS.

**25.** Dispositif selon la revendication 24, caractérisé en ce que les dispositifs (60, 62) de mesure du volume du ventricule gauche comportent des dispositifs pour l'exécution d'un grand nombre de mesures de volume pendant 40 msec.

**26.** Dispositif selon l'une des revendications 16 à 25, caractérisé en ce qu'il comporte des dispositifs pour la mesure de la pression sanguine systolique et diastolique.

**27.** Dispositif selon l'une des revendications 16 à 26, caractérisé en ce qu'il comporte des dispositifs (20) pour le calcul de l'indice de puissance cardiaque en tant que pente de la courbe d'adaptation par régression, calculée par la méthode des moindres carrés, de la totalité de la série de valeurs instantanées de puissance jusqu'à un point de puissance maximale, avec élimination des points dont la valeur est située à l'extérieur de la plage de variance qui correspond aux autres points.

**28.** Dispositif selon l'une des revendications 16 à 27, caractérisé en ce qu'il présente en outre un détecteur (40) d'ondes pulsées et un processeur (42) d'ondes pulsées qui fonctionnent de telle sorte que les influences des perturbations provoquées par des mouvements soient éliminées.

**29.** Dispositif selon la revendication 28, caractérisé en ce que les dispositifs pour la détection de l'arrivée des ondes de pression cardiaque en un emplacement prédéterminé d'une artère, de préférence un emplacement de l'artère du bras, sont constitués par un détecteur de déplacement de la paroi artérielle par effet Doppler sur ultrasons.

**30.** Dispositif selon la revendication 28, caractérisé en ce que les dispositifs pour la détection de l'arrivée des ondes de pression cardiaque en un emplacement prédéterminé d'une artère, de préférence un emplacement de l'artère du bras, sont constitués par un détecteur de flux sanguin par effet Doppler sur ultrasons.

**31.** Dispositif selon l'une des revendications 16 à 30, caractérisé en ce qu'il présente des dispositifs pour l'élimination des influences perturbatrices des mouvements, qui comportent un porte-détecteur Doppler et des dispositifs pour le rejet des basses fréquences du spectre de décalage audio Doppler.

**32.** Dispositif selon la revendication 31, caractérisé en ce que le détecteur d'ultrasons à effet Doppler (convertisseur) (80, 200) est maintenu par une bande de bras qui présente un porte-convertisseur (81, 201) ajustable qui est fixé sur une courroie de fixation (83, 205) ajustable.

**33.** Dispositif selon l'une des revendications 30 à 32, caractérisé en ce que le détecteur d'ultrasons à effet Doppler (convertisseur) (80, 200) est configuré comme emballage plat à cristaux Doppler qui présentent par rapport à l'horizontale un angle d'inclinaison fixe qui vaut typiquement 30°.

**34.** Dispositif selon la revendication 28, caractérisé en ce que le processeur d'ondes pulsées (42) présente un filtre passe-haut (124) qui sépare les hautes fréquences du signal audio, ainsi qu'un convertisseur courant continu d'amplitude RMS (125) qui mesure l'énergie du spectre à haute fréquence et convertit la totalité de la plage RMS (valeur en moyenne quadratique) en une tension continue proportionnelle.

# HERZLEISTUNGSINDEXMONITOR

```
    28              26              24              22
┌──────────┐   ┌──────────┐   ┌──────────┐   ┌──────────┐
│ Massen-  │   │ Hart-    │   │ Herzlei- │   │Anzeige für│
│ speicher │   │kopiervor-│   │ stungs-  │   │physiolo- │
│          │   │richtung  │   │indexan-  │   │gische Daten│
│          │   │          │   │zeige     │   │          │
└──────────┘   └──────────┘   └──────────┘   └──────────┘
```

```
                                                        62              60
                                               ┌──────────┐   ┌──────────┐
                                               │ GAMMA-   │   │          │
     40            42                          │ Kamera   │←──│ GAMMA-   │
┌──────────┐  ┌──────────┐   44                │ CPU      │   │ Kamera   │
│ Impuls-  │  │ Impuls-  │ ┌─────┐  ┌──────────────┐└──────────┘   └──────────┘
│wellenform│→ │wellenform│→│ A/D │→ │ MICROCOMPUTER │
│sensor    │  │prozessor │ └─────┘  │              │   ┌──────────┐
└──────────┘  └──────────┘          │              │←──│ R-Wellen │
                                    └──────────────┘   │Detektor und│
                                                       │Trigger-Generator│
                                                       └──────────┘
```

```
┌────────────────────────────────────────────────────────┐
│   Eingriffsfreier        / Manschettensperr-           │
│   Blutdruckmonitor       / drucksteuereinheit          │
│                                                         │
│   ┌──────────┐ 32          ┌──────────┐ 36             │
│   │ Druckpro-│             │ Pumpen-  │                │
│   │ zessor   │             │ steuerung│                │
│   └──────────┘             └──────────┘                │
│        ↑                        ↓                      │
│   ┌──────────┐ 34          ┌──────────┐ 37             │
│   │ Druckum- │             │ pneuma-  │                │
│   │ former   │             │ tische   │                │
│   │          │             │ Pumpe    │                │
│   └──────────┘             └──────────┘                │
└────────────────────────────────────────────────────────┘
                        38
                  ┌──────────┐
                  │ Man-     │
                  │ schette  │
                  └──────────┘
```

FIG. 1

EP 0 420 085 B1

FIG. 2

GAMMA-Kamera
Minicomputer

Impulswellenformprozessor

EKG
R-Wellen

MIBP/CPC

EP 0 420 085 B1

Fig 3A

Fig 3B

Fig 3C

Fig. 4A

Fig. 4B

Fig. 4C

FIG. 5

FIG 6A

FIG 6B

FIG 6C

VOL.

T

αRS

P

T

Leistung

CPI

T

25

FIG. 7

FIG.8

**DOPPLER-PROZESSOR**

Lautsprecher

123 — Leistungs-verstärker

Volumen

122 — Audio-Bandpass 58-15,000Hz

121 — Phasen-Shift-Board

120 — P-94A Bidirektionaler Doppler-Antrieb

5MHz Fühler

AUDIO

124 — Hochpass-Filter 1400 Hz

125 — RMS-DC-Umformer

126 — programmierbare Verstärkungs-regelung

127 — Isolations-puffer

zu A/D

Doppler AUS

FIG. 9

FIGUR 10a

FIGUR 10 b

**8 BITS** — **101** — **8 BITS** — **102** — **6 BITS** — **103** — **104** — **105**

Taktpuls → parallele Schnittstelle → Taktpuls → 8 BIT Verriegelung → D/A-Wandler | 0÷2,5V 64 Schritte → spannungsgesteuerte Stromquelle → Kondensatorentladung

Ladung/Entladung

BOSCH EBM 502D

Befehlsdekoder 106

Aufblaspumpe ← Aufblasen

Kondensator mit niedriger Leckage 108

Ladung/Entladung Analogschalter 107

SB

Schnellablaßwert ← Schnellfreigabeschaltung 110 ← Überdruckschutz 111

Kondensator Aufladung und Komparator 109

Ablaßwert ← Steuerung für Ventil für langsames Ablassen ← Spannungskomparator 112

OFFSET-Korrektur 113 | zum A/D-Wandler

Druckumformer ← tatsächlicher Druck

FIG. 11

EP 0 420 085 B1

31

FIGUR 12 a

FIGUR 12 b